# EUROPEAN PATENT SPECIFICATION

(11) **EP 3 234 198 B1**
(45) Date of publication and mention of the grant of the patent: **03.04.2024**
(21) Application number: 15871217.4
(22) Date of filing: 18.12.2015
(51) Int. Cl.: C12Q 1/6874, C12Q 1/6825

(54) **CALIBRATION PANELS AND METHODS FOR DESIGNING THE SAME**
KALIBRIERPANEL UND VERFAHREN ZUM ENTWURF DAVON
PANNEAUX D'ÉTALONNAGE ET PROCÉDÉS POUR LEUR CONCEPTION

(30) Priority: 18.12.2014 US 201462093754 P
(43) Date of publication of application: 25.10.2017
(73) Proprietor: Life Technologies Corporation, Carlsbad, CA 92008 (US)
(72) Inventor: MOZHAYSKIY, Vadim, Carlsbad, CA 92008 (US); FU, Yutao, Carlsbad, CA 92008 (US); HUBBELL, Earl, Carlsbad, CA 92008 (US)
(74) Representative: Ziermann, Oliver
(86) International application number: PCT/US2015/066836
(87) International publication number: WO 2016/100895

(56) References cited:
- WO-A1-2014/130388
- WO-A2-2010/028366
- US-A1- 2010 227 321
- US-A1- 2011 065 588
- US-A1- 2012 197 623
- US-A1- 2013 060 482
- US-A1- 2013 090 860
- US-A1- 2014 235 457
- US-A1- 2014 316 716
- US-A1- 2014 336 063
- PAVEL SKUMS ET AL: "Efficient error correction for next-generation sequencing of viral amplicons", BMC BIOINFORMATICS, BIOMED CENTRAL, LONDON, GB, vol. 13, no. Suppl 10, 25 June 2012 (2012-06-25), page S6, XP021094804, ISSN: 1471-2105, DOI: 10.1186/1471-2105-13-S10-S6
- S. J. SALIPANTE ET AL: "Performance Comparison of Illumina and Ion Torrent Next-Generation Sequencing Platforms for 16S rRNA-Based Bacterial Community Profiling", APPLIED AND ENVIRONMENTAL MICROBIOLOGY, vol. 80, no. 24, 26 September 2014 (2014-09-26), pages 7583-7591, XP055330453, US ISSN: 0099-2240, DOI: 10.1128/AEM.02206-14
- FLORA ET AL.: 'Complete sequencing of mono-deprotonated peptide nucleic acids by sustained off-resonance irradiation collision-induced dissociation' J AM SOC MASS SPECTROM vol. 12, no. 7, 01 July 2001, pages 805 - 809, XP004246873 DOI: 10.1016/S1044-0305(01)00259-8

## Description

### RELATED APPLICATION(S)

This application claims priority to U.S. Provisional Application No. 62/093,754 filed December 18, 2014.

### FIELD

This application generally relates to calibration panels and methods for designing the same. More specifically, the application relates to panels of amplicons for homopolymer calibration or recalibration for use with nucleic acid sequencing data and methods for preparing the same.

### BACKGROUND

Nucleic acid sequencing data may be obtained in various ways, including using next-generation sequencing systems, for example, the Ion PGM^{™} and Ion Proton^{™} systems implementing Ion Torrent^{™} sequencing technology (see, e.g., U.S. Pat. No. 7,948,015 and U.S. Pat. Appl. Publ. Nos. 2010/0137143, 2009/0026082, and 2010/0282617. In some cases, such nucleic acid sequencing data may be processed and/or analyzed to obtain base calls using one or more calibration or recalibration processes. Such calibration or recalibration processes may be based on measurement values obtained for randomly selected subsets of nucleic acid templates undergoing sequencing. In some cases, a random selection of nucleic acid templates may result in subsets of nucleic acid templates that lack sufficient representation of long homopolymers. Thus, a desire exists for new and improved methods for designing or selecting sets of amplicons that improve calibration or recalibration processes.

US 2014/316716 A1 describes a method of exposing template polynucleotide strands, sequencing primers, and polymerase to flows of nucleotide species. A series of measured intensity values is obtained and a training subset is randomly selected therefrom. A series of base calls is generated using an aligner. Intensity value thresholds and parameters of a linear transformation corresponding to different homopolymer lengths and nucleotide species are determined and a series of base calls corresponding to the series of measured intensity values is generated using at least some of the parameters of a linear transformation. The series of base calls corresponding to the plurality of series of measured intensity values are recalibrated using at least some of the intensity value thresholds.
US 2013/060482 A1 describes a method for nucleic acid sequencing that includes receiving a plurality of observed or measured signals indicative of a parameter observed or measured for a plurality of defined spaces, determining, for at least some of the defined spaces, whether the defined space comprises one or more sample nucleic acids, processing, for at least some of the defined spaces, the observed or measured signal to improve a quality of the observed or measured signal, generating, for at least some of the defined spaces, a set of candidate sequences of bases for the defined space using one or more metrics adapted to associate a score or penalty to the candidate sequences of bases and selecting the candidate sequence leading to a highest score or a lowest penalty as corresponding to the correct sequence for the one or more sample nucleic acids in the defined space.
US 2012/197623 A1 describes methods for nucleic acid sequence analysis that can analyze data indicative of natural by-products of nucleotide incorporation events without the need for exogenous labels or dyes to identify nucleic acid sequences of interest. In particular, these methods can process such data to align fragments of the nucleic acids of interest analyzed using an addition sequencing technique, as occurs with the use of nucleotide flows.
WO 2010/028366 A2 describes s system for performing quality control for nucleic acid sample sequencing, that comprises a set of solid supports, each solid support having attached thereto a plurality of nucleic acid sequences, wherein the set comprises plural groups of solid supports and each group contains solid supports having the same nucleic acid sequences attached thereto, wherein the nucleic acid sequences of each group differ from each other, and wherein the nucleic acid sequences are synthetically derived.
In XP021094804 (Pavel Skums et. al.) error correction algorithms optimized for viral amplicons are described: k-mer-based error correction (KEC) and empirical frequency threshold (ET).
In XP055330453 (S.J. Salipante et. al.) the performances of two common sequencing platforms, Illumina MiSeq and Ion Torrent Personal Genome Machine, are compared for bacterial community profiling by 16S rRNA-based amplicon sequencing.
US 2014/235457 A1 describes methods for sequencing polynucleotides and detecting reactions and binding events involving other biological molecules. The systems and methods may employ chamber-free devices and nano-sensors to detect or characterize such reactions in high-throughput.

### BRIEF DESCRIPTION OF THE DRAWINGS

The accompanying drawings, which are incorporated into and form a part of the specification, illustrate one or more exemplary embodiments and serve to explain the principles of various exemplary embodiments. The drawings are exemplary and explanatory only and are not to be construed as limiting or restrictive in any way.
FIG. 1 illustrates an exemplary system for nucleic acid sequencing and/or analysis.
FIG. 2 illustrates exemplary components of an apparatus for nucleic acid sequencing.
FIG. 3 illustrates an exemplary flow cell for nucleic acid sequencing.
FIG. 4 illustrates an exemplary computer system for performing nucleic acid sequencing.
FIG. 5 illustrates an exemplary process for label-free, pH-based sequencing.
FIG. 6 illustrates an exemplary method for performing nucleic acid sequencing using a homopolymer calibration panel.
FIG. 7 illustrates an exemplary method for preparing a homopolymer calibration panel.
FIG. 8 shows homopolymer representation statistics for an exemplary recalibration panel.
FIGS. 9A and 9B illustrate exemplary distributions of amplicon length for a two-exome sequencing run.
FIGS. 10A and 10B show exemplary distributions of GC content for a two-exome sequencing run.
FIGS. 11A and 11B show exemplary distributions of coverage, expressed as percentage of median coverage, for a two-exome sequencing run.
FIGS. 12A and 12B illustrate exemplary distributions of percentage forward end-to-end for a two-exome sequencing run.
FIGS. 13A and 13B illustrate exemplary distributions of percentage reverse end-to-end for a two-exome sequencing run.
FIGS. 14A and 14B illustrate exemplary distributions of strand bias.
FIG. 15 illustrates an exemplary plot showing the correlation between forward and reverse end-to-end coverage.
FIG. 16 illustrates an exemplary plot showing the estimated per-homopolymer coverage relative to the median panel coverage.
FIG. 17 includes an exemplary table, which lists a chromosome, insert start, insert end, and insert for each of the amplicons of the exemplary recalibration panel of FIG. 8, according to one embodiment.

### SUMMARY

A method is provided for nucleic acid sequencing, comprising: (a) disposing a plurality of template polynucleotide strands in a plurality of defined spaces of a sensor array, the template polynucleotide strands comprising a predetermined set of homopolymer recalibration template polynucleotide strands; (b) exposing a plurality of the template polynucleotide strands, including the predetermined set of homopolymer recalibration template polynucleotide strands in the defined spaces, to a series of flows of nucleotide species flowed according to a predetermined ordering; and (c) determining sequence information for the plurality of the template polynucleotide strands, including the predetermined set of homopolymer recalibration template polynucleotide strands in the defined spaces, based on the flows of nucleotide species, to generate a plurality of sequencing reads corresponding to the template polynucleotide strands. The predetermined set of homopolymer recalibration template polynucleotide strands comprises amplicon sequences that together comprise at least a predetermined minimal threshold number of homopolymers of each homopolymer length between a predetermined minimal homopolymer length and a predetermined maximal homopolymer length for one or more of homopolymer types A, T, C, and G. In one embodiment of the method, the predetermined minimal homopolymer length and the predetermined maximal homopolymer length is for each of homopolymer types A, T, C, and G.

In such a method, the minimal threshold number may be 10, for example. Alternately or in addition, the minimal threshold number may be 25, for example. Alternately or in addition, the minimal threshold number may be 50, for example. The homopolymer recalibration template polynucleotide strands may comprise amplicon sequences that are comprised in high-confidence regions of a reference genome (e.g., NIST NA12878) with no variants, for example. The homopolymer recalibration template polynucleotide strands may comprise amplicon sequences that include, at most, one homopolymer of length 6, 7, 8, 9, or 10 per amplicon sequence, for example. The homopolymer recalibration template polynucleotide strands may comprise amplicon sequences having a minimal distance of 7 bases between any homopolymers of length 4, 5, 6, 7, 8, 9, or 10, for example. The homopolymer recalibration template polynucleotide strands may comprise amplicon sequences that do not overlap. The predetermined minimal homopolymer length may be 5, for example. The predetermined maximal homopolymer length may be 10, for example.

A system is provided, including: a plurality of template polynucleotide strands disposed in a plurality of defined spaces of a sensor array, the template polynucleotide strands comprising a predetermined set of homopolymer recalibration template polynucleotide strands, wherein the predetermined set of homopolymer recalibration template polynucleotide strands comprises amplicon sequences that together comprise at least a predetermined minimal threshold number of homopolymers of each homopolymer length between a predetermined minimal homopolymer length and a predetermined maximal homopolymer length for one or more of homopolymer types A, T, C, and G; a machine-readable memory; and a processor configured to execute machine-readable instructions, which, when executed by the processor, cause the system to perform a method for nucleic acid sequencing, comprising: (a) exposing the plurality of the template polynucleotide strands, including the predetermined set of homopolymer recalibration template polynucleotide strands in the defined spaces, to a series of flows of nucleotide species flowed according to a predetermined ordering; and (b) determining sequence information for the plurality of the template polynucleotide strands, including the predetermined set of homopolymer recalibration template polynucleotide strands in the defined spaces, based on the flows of nucleotide species, e.g., to generate a plurality of sequencing reads corresponding to the template polynucleotide strands.

In such a system, the homopolymer recalibration template polynucleotide strands may include amplicon sequences that are comprised in high-confidence regions of a reference genome (e.g., NIST NA12878) with no variants. In one embodiment of the system, the predetermined minimal homopolymer length and the predetermined maximal homopolymer length is for each of homopolymer types A, T, C, and G.

According to an exemplary embodiment, there is provided a method for preparing a homopolymer recalibration panel, comprising: extracting, from a set of amplicons used in sequencing-by-synthesis, a set of candidate amplicons satisfying a first set of criteria, wherein the first set of criteria includes amplicons known to belong to high-confidence regions of a reference genome with no variants; and selecting, from the set of candidate amplicons, a reduced set of amplicons satisfying a second set of criteria, wherein the second set of criteria includes amplicons that together comprise at least a minimal threshold number of homopolymers of each homopolymer length between a predetermined minimal homopolymer length and a predetermined maximal homopolymer length for one or more of homopolymer types A, T, C, and G.

In such a method, the minimal threshold number may be 10, for example. Alternately or in addition, the minimal threshold number may be 25, for example. Alternately or in addition, the minimal threshold number may be 50, for example. The reference genome with no variants may be NIST NA12878, for example. The predetermined minimal homopolymer length and the predetermined maximal homopolymer length is for each of homopolymer types A, T, C, and G.

The reduced set of amplicons may comprise at most one homopolymer of length 6, 7, 8, 9, or 10 per amplicon, for example. The reduced set of amplicons may comprise amplicons having a minimal distance of 7 bases between any homopolymers of length 4, 5, 6, 7, 8, 9, or 10, for example. The reduced set of amplicons may comprise amplicons that do not overlap. The predetermined minimal homopolymer length may be 5, for example. The predetermined maximal homopolymer length may be 10, for example.

The method may further comprise determining underrepresented homopolymers of the set of candidate amplicons; and augmenting the set of candidate amplicons with a predetermined number of the underrepresented homopolymers. The method may further comprise disposing the reduced set of amplicons in a plurality of defined spaces of a sensor array. The method may also further comprise exposing the reduced set of amplicons to a series of glows of nucleotide species flowed according to a predetermined ordering; and determining sequence information for the reduced set of amplicons based on the flows of nucleotide species, to generate a plurality of sequencing reads corresponding to the reduced set of amplicons.

According to an exemplary embodiment, there is provided a homopolymer recalibration panel, comprising: a set of candidate amplicons extracted from a set of amplicons used in sequencing-by-synthesis, wherein the amplicons in the set of candidate amplicons: (a) are known to belong to high-confidence regions of a reference genome with no variants; and (b) together comprise at least a minimal threshold number of homopolymers of each homopolymer length between a predetermined minimal homopolymer length and a predetermined maximal homopolymer length for one or more of homopolymer types A, T, C, and G.

In various embodiments, a panel comprising amplicons with predetermined base sequences as described herein may be synthesized using any suitable nucleic acid synthesis methods known in the art.

In some embodiments, recalibration may include a single-pass calibration process in which a recalibration engine changes or modifies a set of default/initial parameters (e.g., homopolymers of various lengths being treated/weighed the same or according to some factory pre-determined set of initial homopolymer-specific parameters or weights). In other embodiments, recalibration may include a multi-pass or iterative process in which previously calibrated or recalibrated parameters may be further changed or modified by the calibration process.

The reference genome with no variants may be NIST NA12878, for example. The minimal threshold number may be 10, 25, or 50, for example. Alternately or in addition, the minimal threshold number may be 25, for example. Alternately or in addition, the minimal threshold number may be 50, for example.

### EXEMPLARY EMBODIMENTS

The following description and the various embodiments described herein are exemplary and explanatory only and are not to be construed as limiting or restrictive in any way. Other embodiments, features, objects, and advantages of the present teachings will be apparent from the description and accompanying drawings, and from the claims.

According to various exemplary embodiments, panels of amplicons for homopolymer calibration or recalibration for use with nucleic acid sequencing data and methods for designing the same, are disclosed herein. Such panels of amplicons may improve downstream processing (including variant calling), since such panels may improve calibration and recalibration of nucleic acid sequencing data and/or reduce certain systematic errors and improve overall sequencing accuracy (especially in the case of long homopolymers).

### Design of Calibration Panel

In various embodiments, a homopolymer calibration panel may be designed to have a substantially uniform representation of homopolymers of various lengths. Homopolymers of relatively short lengths (e.g., 2, 3, and 4 bases) may be well represented in a sufficiently large set of sufficiently long sequences selected using any suitable arbitrary or random approach. However, homopolymers of relatively long lengths (e.g., 5, 6, 7, 8, 9, and 10, or more) are naturally rarer than homopolymers of relatively short lengths. Thus, homopolymers of relatively long lengths may be insufficiently represented (or at least under-represented compared with shorter homopolymers) among sequences selected using any suitable arbitrary or random approach.

In an embodiment, a set of sequences containing a desired uniform representation across homopolymers may be defined by computationally and combinatorially populating a set of sequences with desired types and quantities of homopolymers. For example, a set of sequences may be populated to include exactly or at least *n(MinL, MaxL, NumT)* homopolymers, each homopolymer having a length between lengths *MinL* and *MaxL* (e.g., each length between *MinL* = 1 and *MaxL* = 10, or each length between *MinL* = 5 and *MaxL* = 10, etc.). The set of sequences may further be populated to include at least one type of nucleotide among *NumT* types of nucleotides (e.g., each type among *NumT* = 4 types A, C, G, and T), where *n* is an integer (e.g., 10, 25, 50, 100, etc.) that may be a function of parameters *MinL, MaxL,* and *NumT.*

In some cases, one or more sets of sequences containing such homopolymers populated computationally and combinatorially may not have been empirically tested and may not be ideally suited for a given underlying sequencing technology. One method to address this may include designing a set of sequences containing a substantially uniform representation across homopolymers using steps including: (1) identifying an initial set of candidate amplicons or oligonucleotides known to function properly on a particular sequencing platform or technology (e.g., a set of amplicons from the Ion AmpliSeq^{™} Exome Panel or any suitable panel used with some given underlying sequencing technology); (2) selecting, from the initial set of candidate amplicons or oligonucleotides, a subset of amplicons or oligonucleotides meeting one or more selection criteria (e.g., one or more minimal numbers of occurrences of homopolymers of certain lengths and types, for example, those having at least 50 (or some suitable integer, such as 10, 25, 50, 75, etc., for example) homopolymers of each length 5, 6, 7, 8, 9, and 10, and/or of each type A, C, G, and T); and (3) augment the subset of amplicons or oligonucleotides with additional amplicons or oligonucleotides comprising a desired number of under-represented homopolymers (e.g., by adding a substantial number of homopolymers of length 9 and/or 10 or other comparatively rare and under-represented length(s)). Augmenting the subset of amplicons or oligonucleotides with additional amplicons or oligonucleotides may help achieve a desired level of representation uniformity across homopolymers.

In an embodiment, a set of sequences containing a substantially uniform representation across homopolymers may be designed using steps including: (1) identifying an initial set of candidate amplicons or oligonucleotides known to have been used with a particular sequencing platform or technology; (2) selecting from the initial set of candidate amplicons or oligonucleotides a subset of amplicons or oligonucleotides that together include all *n*-mers up to a predetermined maximal homopolymer length (e.g., up to *n* = 5, 6, 7, 8, 9, or 10) for bases A, C, G, and T with a predetermined minimum of *n*-mers of each length and/or type of nucleotide (e.g., at least 10, 25, 50, 75, or more, of each length and/or type); and (3) performing an empirically-based pruning or refinement selection to reduce the impact on throughput. Such pruning or refinement selection may include minimizing the number of amplicons or oligonucleotides by selecting amplicons that have several *n*-mers but maintaining the quality of the selected amplicons (e.g., in an example further discussed below, a final panel may have 384 amplicons, a 23% reduction from a starting point of 500 candidate amplicons, where 500 may be the product of 50 n-mers times 4 bases times 5 (for n = 6, 7, 8, 9, and 10, with shorter n-mers being automatically included), divided by 2 (for strands that produce complementary homopolymers)).

In various embodiments, a homopolymer calibration panel may be designed using steps including: (1) identifying an initial set of candidate amplicons or oligonucleotides that are known to have been used with a particular sequencing platform or technology (e.g., a substantial number, such as 300, 400, 500, or more, amplicons or oligonucleotides from the Ion AmpliSeq^{™} Exome Panel or any suitable panel used with some given underlying sequencing technology) and that are inside high-confidence regions of a reference genome (e.g., NIST NA12878) with no variants so that the real homopolymer length(s) may be known; (2) selecting, from the initial set of candidate amplicons or oligonucleotides, a subset of amplicons or oligonucleotides that together include a predetermined minimum of n-mers of each homopolymer length from a predetermined minimal length to a predetermined maximal length for all bases A, C, G, and T; (3) filtering out amplicons or oligonucleotides that violate one or more of the following constraints: (a) having more than one same-base n-mer with *n* = 6 or more, (b) having more than a minimal separation of 7 bases between *n-*mers with n = 4 or more bases to obviate or reduce additional errors and de-phasing that may be introduced with neighboring homopolymers (except that several G or C n-mers may be on the same strand if they are separated by at least 7 bases and at least 3 bases from A/T n-mers, given that long C and G homopolymers may be rare and GC-rich regions may be particularly difficult to sequence), (c) having an overlap with another amplicon or oligonucleotide in the set, and (d) having a homopolymer of length longer than 10; and (4) if desired to achieve a determined or selected level of representation uniformity across homopolymers, augment the subset of amplicons or oligonucleotides with additional amplicons or oligonucleotides comprising a desired number of under-represented homopolymers (e.g., by adding a substantial number of G and C homopolymers of length 9 and/or 10 to represent problematic homopolymers in the set).

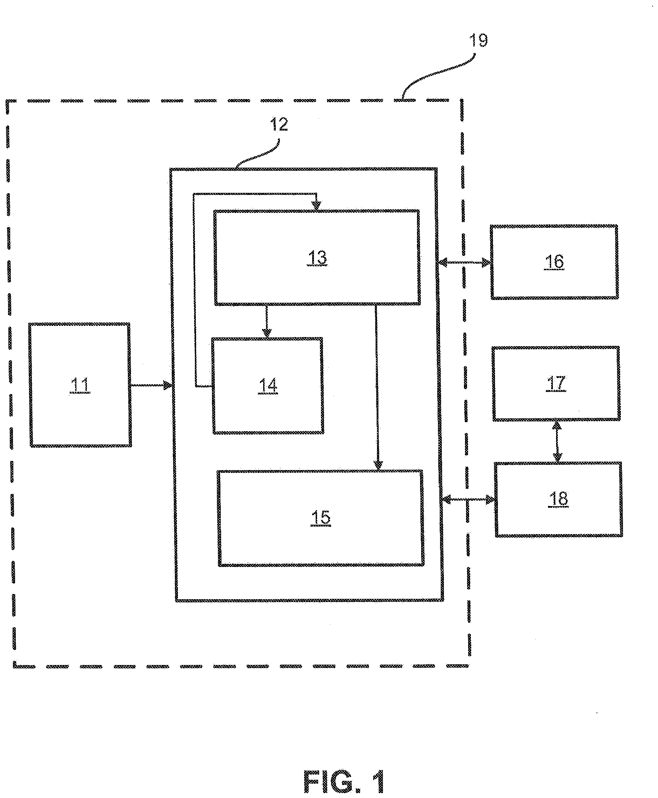

FIG. 1 illustrates an exemplary system for nucleic acid sequencing and/or analysis. The system includes an apparatus or sub-system for nucleic acid sequencing and/or analysis 11, a computing server/node/device 12 including a base calling engine 13, a recalibration engine 14, a post-processing engine 15, and a display 16, which may be internal and/or external. The apparatus or sub-system for nucleic acid sequencing and/or analysis 11 may be any type of instrument that can generate nucleic acid sequence data from nucleic acid samples, for example, a nucleic acid sequencing instrument, a real-time/digital/quantitative PCR instrument, a microarray scanner, etc. The nucleic acid samples may include amplicons for calibration or recalibration as further described herein. The computing server/node/device 12 may include a workstation, mainframe computer, distributed computing node (part of a "cloud computing" or distributed networking system), personal computer, mobile device, etc. The base calling engine 13 may include any suitable base caller and may be configured to include various signal/data processing modules that may be configured to receive signal/data from the apparatus or sub-system for nucleic acid sequencing and/or analysis 11 and perform various processing steps, for example, conversion from flow space to base space, determination of base calls for some or the entirety of a sequencing data set, and/or determination of base call quality values. In an embodiment, the base calling engine 13 may implement one or more features described in Davey et al, U.S. Pat. Appl. Publ. No.2012/0109598, published on May 3, 2012, and/or Sikora et al., U.S. Pat. Appl. Publ. No. 2013/0060482, published on March 7, 2013. The base calling engine 13 may also include a mapping or alignment module for mapping or aligning reads to a reference sequence or genome, which may include a whole/partial genome, a whole/partial exome, etc. In an embodiment, the mapping or alignment module may include any suitable aligner, including the Torrent Mapping Alignment Program (TMAP), for example. The recalibration engine 14 may be configured to recalibrate base calls or related intensity values or parameters based on an analysis of base calling and alignment performed by the base calling engine 13. Recalibrated base calls or related intensity values, thresholds, or parameters may be fed back into the base calling engine 13 for improving the accuracy of base calls. In an embodiment, the recalibration engine 14 may implement one or more features described in Jiang et al, U.S. Pat. Appl. Publ. No. 2014/0316716, published on October 23, 2014. The exemplary system may also include a client device terminal 17, which may include a data analysis API or module and may be communicatively connected to the computing server/node/device 12 via a network connection 18 that may be a "hardwired" physical network connection (e.g., Internet, LAN, WAN, VPN, etc.) or a wireless network connection (e.g., Wi-Fi, WLAN, etc.). The post-processing engine 15 may be configured to include various signal/data processing modules that may be configured to make variant calls and apply post-processing to variant calls, which may include annotating various variant calls and/or features, converting data from flow space to base space, filtering of variants, and/or formatting the variant data for display or use by client device terminal 17. Variant calls may be made using any suitable variant caller, including the Germ-Line Variant Caller and the Torrent Variant Caller (TVC) Plug-ins for Ion Torrent^{™} sequencing technology. In an embodiment, the variant caller may implement one or more features described in Hubbell et al., U.S. Pat. Appl. No. 2014/0296080, published on October 2, 2014, which is incorporated by reference herein in its entirety. In an embodiment, the apparatus or sub-system for nucleic acid sequencing and/or analysis 11 and the computing server/node/device 12 may be integrated into a single instrument or system comprising components present in a single enclosure 19. The client device terminal 17 may be configured to communicate information to and/or control the operation of the computing server/node/device 12 and its modules and/or operating parameters.

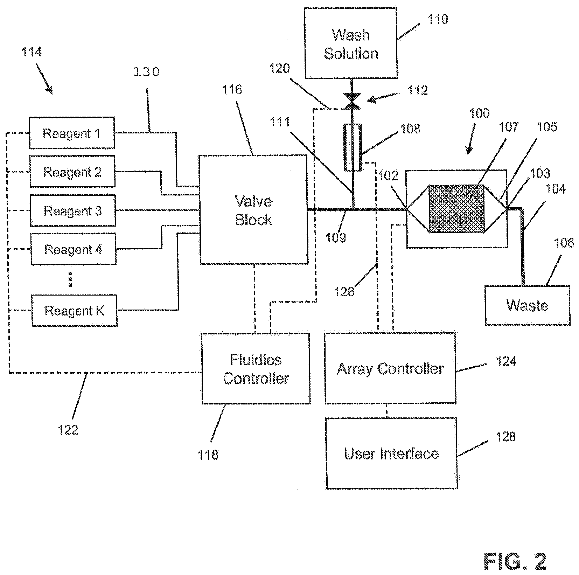

FIG. 2 illustrates exemplary components of an apparatus for nucleic acid sequencing. Such an apparatus could be used as apparatus or sub-system for nucleic acid sequencing and/or analysis 11 of FIG. 1. The components include a flow cell and sensor array 100, a reference electrode 108, a plurality of reagents 114, a valve block 116, a wash solution 110, a valve 112, a fluidics controller 118, lines 120/122/126, passages 104/109/111, a waste container 106, an array controller 124, and a user interface 128. The flow cell and sensor array 100 includes an inlet 102, an outlet 103, a microwell array 107, and a flow chamber 105 defining a flow path of reagents over the microwell array 107. The reference electrode 108 may be of any suitable type or shape, including a concentric cylinder with a fluid passage or a wire inserted into a lumen of passage 111. The reagents 114 may be driven through the fluid pathways, valves, and flow cell by pumps, gas pressure, or other suitable methods, and may be discarded into the waste container 106 after exiting the flow cell and sensor array 100. The reagents 114 may, for example, contain dNTPs to be flowed through passages 130 and through the valve block 116, which may control the flow of the reagents 114 to flow chamber 105 (also referred to herein as a reaction chamber) via passage 109. The system may include a reservoir for containing a wash solution 110 that may be used to wash away dNTPs, for example, that may have previously been flowed. The microwell array 107 may include an array of defined spaces (e.g., microwells), for example, wherein the array may be operationally associated with a sensor array so that each microwell may be associated with a sensor suitable for detecting an analyte or reaction property of interest. The defined spaces may include nucleic acid samples, which may include amplicons for calibration or recalibration as further described herein. The microwell array 107 may be integrated with the sensor array as a single device or chip. The array controller 124 may provide bias voltages, timing, and/or control signals to the sensor, and collect and/or process output signals. The user interface 128 may display information from the flow cell and sensor array 100 as well as instrument settings and controls, and allow a user to enter or set instrument settings and controls. The valve 112 may be shut to prevent any wash solution 110 from flowing into passage 109 as the reagents are flowing. Although the flow of wash solution may be stopped, there may still be uninterrupted fluid and electrical communication between the reference electrode 108, passage 109, and the microwell array 107. The fluidics controller 118 may be programmed to control driving forces for flowing reagents 114 and to control the operation of valve 112 and valve block 116 to deliver reagents to the flow cell and sensor array 100 according to a predetermined reagent flow ordering.

In this application, "defined space" may refer to any space (which may be in one, two, or three dimensions) in which at least some of a molecule, fluid, and/or solid can be confined, retained, and/or localized. A space may be a predetermined area (which may be a flat area) or volume, and may be defined, for example, by a depression or a micro-machined well in or associated with a microwell plate, microtiter plate, microplate, or a chip, or by isolated hydrophobic areas on a generally hydrophobic surface. Defined spaces may be arranged as an array, which may be a substantially planar one-dimensional or two-dimensional arrangement of elements, including sensors or wells. Defined spaces, whether arranged as an array or in some other configuration, may be in electrical communication with at least one sensor to allow detection or measurement of one or more detectable or measurable parameters or characteristics. The sensors may convert changes in the presence, concentration, or amounts of reaction by-products (or changes in ionic character of reactants) into an output signal, which may be registered electronically, for example, as a change in a voltage level or a current level. In one embodiment, the output signal and/or change in voltage or current level, in turn, may be processed to extract information or signal about a chemical reaction or desired association event, for example, a nucleotide incorporation event and/or a related ion concentration (e.g., a pH measurement). The sensors may include at least one ion sensitive field effect transistor ("ISFET") and/or chemically sensitive field effect transistor ("chemFET").

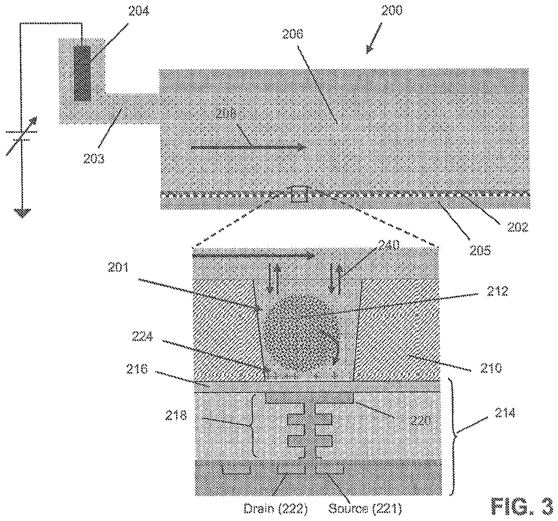

FIG. 3 illustrates an exemplary flow cell for nucleic acid sequencing. The flow cell 200 includes a microwell array 202, a sensor array 205, and a flow chamber 206 in which a reagent flow 208 may move across a surface of the microwell array 202 and/or over open ends of microwells in the microwell array 202. The flow of reagents (e.g., nucleotide species) can be provided in any suitable manner, including delivery by pipettes, or through tubes or passages connected to a flow chamber. A microwell 201 in the microwell array 202 may have any suitable volume, shape, and aspect ratio. A sensor 214 in the sensor array 205 may be an ISFET or a chemFET sensor with a floating gate 218 having a sensor plate 220 separated from the microwell interior by a passivation layer 216, and may be predominantly responsive to (and generate an output signal related to) an amount of charge 224 present on the passivation layer 216 opposite of the sensor plate 220. Changes in the amount of charge 224 may cause changes in a current between a source 221 and a drain 222 of the sensor 214, which may be used directly to provide a current-based output signal, or indirectly with additional circuitry to provide a voltage output signal. Reactants, wash solutions, and other reagents may move into microwells primarily by diffusion 240. One or more analytical reactions to identify or determine characteristics or properties of an analyte of interest may be carried out in one or more microwells of the microwell array 202. Such reactions may directly or indirectly generate by-products that affect the amount of charge 224 adjacent to the sensor plate 220. In an embodiment, a reference electrode 204 may be fluidly connected to the flow chamber 206 via a flow passage 203. In an embodiment, the microwell array 202 and the sensor array 205 may together form an integrated unit forming a bottom wall or floor of the flow cell 200. In an embodiment, one or more copies of an analyte may be attached to a solid phase support 212, which may include microparticles, nanoparticles, beads, or gels, and may be solid and porous, for example. The analyte may include one or more copies of a nucleic acid analyte, which may include nucleic acid samples. Exemplary samples may include amplicons for calibration or recalibration, which may be obtained using any suitable technique, as further described herein.

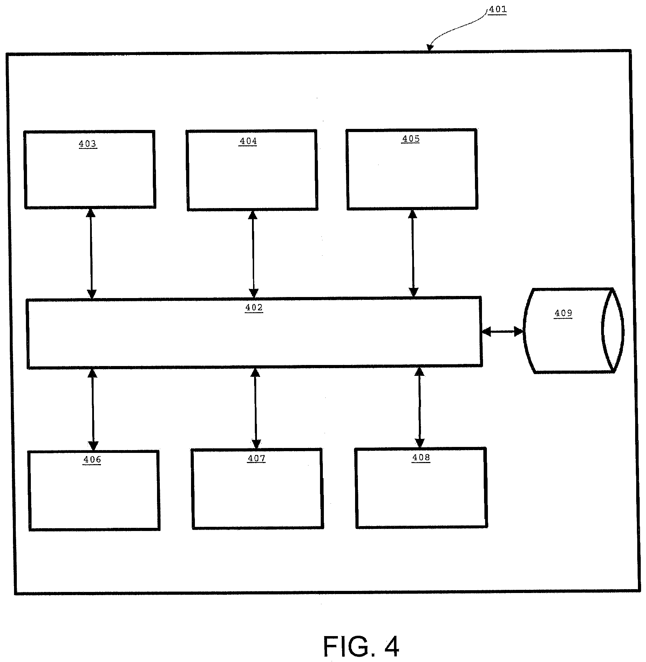

FIG. 4 illustrates an exemplary computer system 401 for performing nucleic acid sequencing. Such a computer system 401 could be used as computing server/node/device 12 of FIG. 1. The computer system 401 may include a bus 402 or other communication mechanism for communicating information, a processor 403 coupled to the bus 402 for processing information, and/or a memory 405 coupled to the bus 402 for dynamically and/or statically storing information. The computer system 401 can also include one or more coprocessors 404 coupled to the bus 402 (e.g., GPUs and/or FPGAs) for performing specialized processing tasks; a display 406 coupled to the bus 402 (e.g., a cathode ray tube (CRT) or liquid crystal display (LCD)) for displaying information to a computer user; an input device 407 coupled to the bus 402 (e.g., a keyboard including alphanumeric and other keys) for communicating information and command selections to the processor 403; a cursor control device 408 coupled to the bus 402 (e.g., a mouse, a trackball, or cursor direction keys) for communicating direction information and command selections to the processor 403 and for controlling cursor movement on display 406; and one or more storage devices 409 coupled to the bus 402 (e.g., a magnetic disk or an optical disk) for storing information and instructions. The memory 405 may include a random access memory (RAM) or other dynamic storage device and/or a read only memory (ROM) or other static storage device. Such an exemplary computer system with suitable software may be used to perform the embodiments described herein. More generally, in various embodiments, one or more features of the teachings and/or embodiments described herein may be performed or implemented using appropriately configured and/or programmed hardware and/or software elements.

Examples of hardware elements may include processors, microprocessors, input(s) and/or output(s) (I/O) device(s) (or peripherals) that are communicatively coupled via a local interface circuit, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), logic gates, registers, semiconductor devices, chips, microchips, chip sets, and so forth. The local interface may include, for example, one or more buses or other wired or wireless connections, controllers, buffers (caches), drivers, repeaters and receivers, etc., to allow appropriate communications between hardware components. A processor may include a hardware device for executing software, particularly software stored in memory. The processor may include any custom made or commercially available processor, a central processing unit (CPU), an auxiliary processor among several processors associated with the computer, a semiconductor based microprocessor (e.g., in the form of a microchip or chip set), a macroprocessor, or generally any device for executing software instructions. A processor can also represent a distributed processing architecture. The I/O devices can include input devices, for example, a keyboard, a mouse, a scanner, a microphone, a touch screen, an interface for various medical devices and/or laboratory instruments, a bar code reader, a stylus, a laser reader, a radiofrequency device reader, etc. Furthermore, the I/O devices also can include output devices, for example, a printer, a bar code printer, a display, etc. Finally, the I/O devices further can include devices that communicate as both inputs and outputs, for example, a modulator/demodulator (modem; for accessing another device, system, or network), a radio frequency (RF) or other transceiver, a telephonic interface, a bridge, a router, etc.

Examples of software may include software components, programs, applications, computer programs, application programs, system programs, machine programs, operating system software, middleware, firmware, software modules, routines, subroutines, functions, methods, procedures, software interfaces, application program interfaces (API), instruction sets, computing code, computer code, code segments, computer code segments, words, values, symbols, or any combination thereof. A software in memory may include one or more separate programs, which may include ordered listings of executable instructions for implementing logical functions. The software in memory may include a system for identifying data streams in accordance with the present teachings and any suitable custom made or commercially available operating system (O/S), which may control the execution of other computer programs such as the system, and provide scheduling, input-output control, file and data management, memory management, communication control, etc.

According to various embodiments, one or more features of teachings and/or embodiments described herein may be performed or implemented using an appropriately configured and/or programmed non-transitory machine-readable medium or article that may store an instruction or a set of instructions that, if executed by a machine, may cause the machine to perform a method and/or operations in accordance with the embodiments. Such a machine may include, for example, any suitable processing platform, computing platform, computing device, processing device, computing system, processing system, computer, processor, scientific or laboratory instrument, etc., and may be implemented using any suitable combination of hardware and/or software. The machine-readable medium or article may include, for example, any suitable type of memory unit, memory device, memory article, memory medium, storage device, storage article, storage medium and/or storage unit, for example, memory, removable or non-removable media, erasable or non-erasable media, writeable or re-writeable media, digital or analog media, hard disk, floppy disk, read-only memory compact disc (CD-ROM), recordable compact disc (CD-R), rewriteable compact disc (CD-RW), optical disk, magnetic media, magneto-optical media, removable memory cards or disks, various types of Digital Versatile Disc (DVD), a tape, a cassette, etc., including any medium suitable for use in a computer. Memory can include any one or a combination of volatile memory elements (e.g., random access memory (RAM, such as DRAM, SRAM, SDRAM, etc.)) and nonvolatile memory elements (e.g., ROM, EPROM, EEROM, flash memory, hard drive, tape, CDROM, etc.). Moreover, memory can incorporate electronic, magnetic, optical, and/or other types of storage media. Memory can have a distributed, clustered, remote, or cloud architecture where various components may be situated remote from one another, and accessed by the processor. The instructions may include any suitable type of code, including source code, compiled code, interpreted code, executable code, static code, dynamic code, encrypted code, etc., implemented using any suitable high-level, low-level, object-oriented, visual, compiled and/or interpreted programming language.

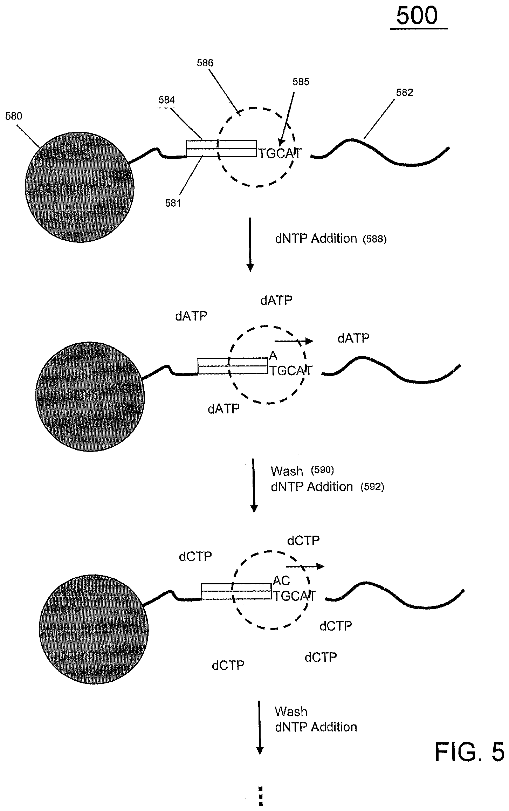

FIG. 5 illustrates an exemplary process 500 for label-free, pH-based sequencing. In one embodiment, a template 582 with sequence 585 and a primer binding site 581 may be attached to a solid phase support 580. The template 582 may include nucleic acid samples comprising amplicons for calibration or recalibration as further described herein. The template 582 may be attached as a clonal population to a solid support (e.g., a microparticle or bead), for example, and may be prepared as disclosed in Leamon et al., U.S. Pat. No. 7,323,305. In an embodiment, the template may be associated with a substrate surface or present in a liquid phase with or without being coupled to a support. A primer 584 and DNA polymerase 586 may be annealed to the template 582 so that the primer's 3' end may be extended by a polymerase, and a polymerase may be bound to such a primer-template duplex (or in close proximity thereof) so that binding and/or extension may take place when dNTPs are added. In one embodiment, step 588 may include adding dNTP (shown as dATP), and the DNA polymerase 586 may incorporate a nucleotide "A" (since "T" may be the next nucleotide in the template 582, and "T" is complementary to the flowed dATP nucleotide). In one embodiment, step 590 may include performing a wash. In one embodiment, step 592 may include adding the next dNTP (shown as dCTP), and the DNA polymerase 586 may incorporate a nucleotide "C" (since "G" may be the next nucleotide in the template 582). More details about pH-based nucleic acid sequencing may be found in U.S. Pat. No.7,948,015 and U.S. Pat. Appl. Publ. Nos. 2010/0137143, 2009/0026082, and 2010/0282617.

In an embodiment, the primer-template-polymerase complex may be subjected to a series of exposures of different nucleotides in a pre-determined sequence or ordering. If one or more nucleotides are incorporated, the signal resulting from the incorporation reaction may be detected. In one embodiment, the nucleotide sequence of the template strand may be determined after repeated cycles of nucleotide addition, primer extension, and/or signal acquisition. The output signals measured throughout this process may depend on the number of nucleotide incorporations. Specifically, in each addition step, the polymerase may extend the primer by incorporating added dNTP only if the next base in the template is complementary to the added dNTP. With each incorporation, a hydrogen ion may be released, and collectively, a population of released hydrogen ions may change a local pH of the respective reaction chamber. The production of hydrogen ions may be monotonically related to the number of contiguous complementary bases (e.g., homopolymers) in the template. Deliveries of nucleotides to a reaction vessel or chamber may be referred to as "flows" of nucleotide triphosphates (or dNTPs). For convenience, a flow of dATP will sometimes be referred to as "a flow of A" or "an A flow," and a sequence of flows may be represented as a sequence of letters, such as "ATGT" indicating "a flow of dATP, followed by a flow of dTTP, followed by a flow of dGTP, followed by a flow of dTTP." The predetermined ordering may be based on a cyclical, repeating pattern including consecutive repeats of a short pre-determined reagent flow ordering (e.g., consecutive repeats of predetermined sequence of four nucleotide reagents, for example, "ACTG ACTG ..."). The predetermined ordering may be based in whole or in part on some other pattern of reagent flows (e.g., any of the various reagent flow orderings discussed in Hubbell et al., U.S. Pat. Appl. Publ. No. 2012/0264621, published October 18, 2012.

In various embodiments, output signals due to nucleotide incorporation may be processed, given knowledge of what nucleotide species were flowed and in what order to obtain such signals. The output signals may be processed to make base calls for the flows and/or to compile consecutive base calls associated with a sample nucleic acid template into a read. A base call may refer to a particular nucleotide identification (e.g., dATP ("A"), dCTP ("C"), dGTP ("G"), or dTTP ("T")). Base calling may include performing one or more signal normalizations, signal phase and signal decay (e.g, enzyme efficiency loss) estimations, signal corrections, and model-based signal predictions. Base calling may also identify or estimate base calls for each flow for each defined space. Any suitable base calling method may be used, including as described in Davey et al, U.S. Pat. Appl. Publ. No. 2012/0109598, published on May 3, 2012, and/or Sikora et al, U.S. Pat. Appl. Publ. No. 2013/0060482, published on March 7, 2013, taking into account that more accurate base callers may yield better results.

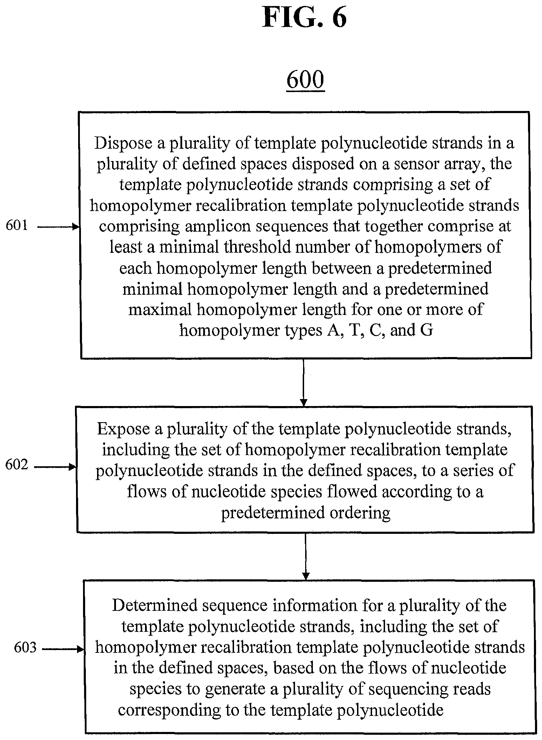

FIG. 6 illustrates an exemplary method 600 for nucleic acid sequencing using a homopolymer calibration panel. In step 601, a plurality of template polynucleotide strands may be disposed in a plurality of defined spaces disposed on a sensor array, the template polynucleotide strands comprising a set of homopolymer recalibration template polynucleotide strands, wherein the homopolymer recalibration template polynucleotide strands may comprise amplicon sequences that together comprise at least a minimal threshold number of homopolymers of each homopolymer length between a predetermined minimal homopolymer length and a predetermined maximal homopolymer length for one or more of homopolymer types A, T, C, and G (e.g., each of homopolymer types A, T, C, and G). In step 602, the plurality of the template polynucleotide strands, including the set of homopolymer recalibration template polynucleotide strands in the defined spaces, may be exposed to a series of flows of nucleotide species flowed according to a predetermined ordering. In step 603, sequence information for a plurality of the template polynucleotide strands, including the set of homopolymer recalibration template polynucleotide strands in the defined spaces, may be determined based on the flows of nucleotide species, e.g., to generate a plurality of sequencing reads corresponding to the template polynucleotide strands.

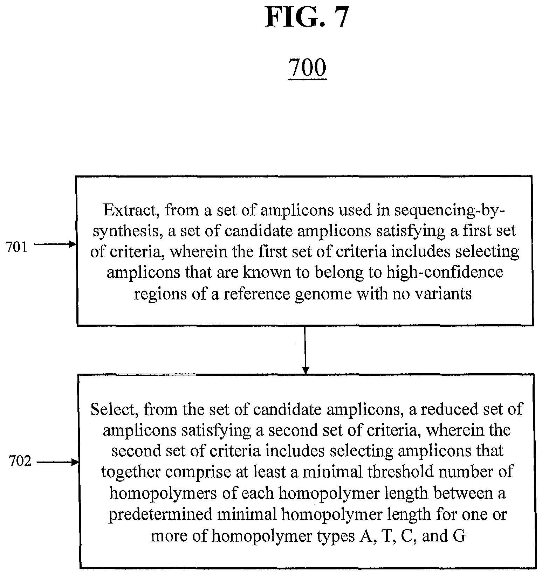

FIG. 7 illustrates an exemplary method 700 for preparing a homopolymer recalibration panel. In one embodiment, the recalibration panel may include amplicons that are 200 base pairs in length. In another embodiment, the recalibration panel may include amplicons longer than 200 base pairs in length (e.g., amplicons that are 600 base pairs in length). In step 701, a set of candidate amplicons satisfying a first set of criteria may be extracted from a set of amplicons used in sequencing-by-synthesis, wherein the first set of criteria may include selecting amplicons known to belong to high-confidence regions of a reference genome with no variants. In step 702, a reduced set of amplicons satisfying a second set of criteria may be selected from the set of candidate amplicons, wherein the second set of criteria may include selecting amplicons that together comprise at least a minimal threshold number of homopolymers of each homopolymer length between a predetermined minimal homopolymer length and a predetermined maximal homopolymer length for one or more of homopolymer types A, T, C, and G (e.g., each of homopolymer types A, T, C, and G).

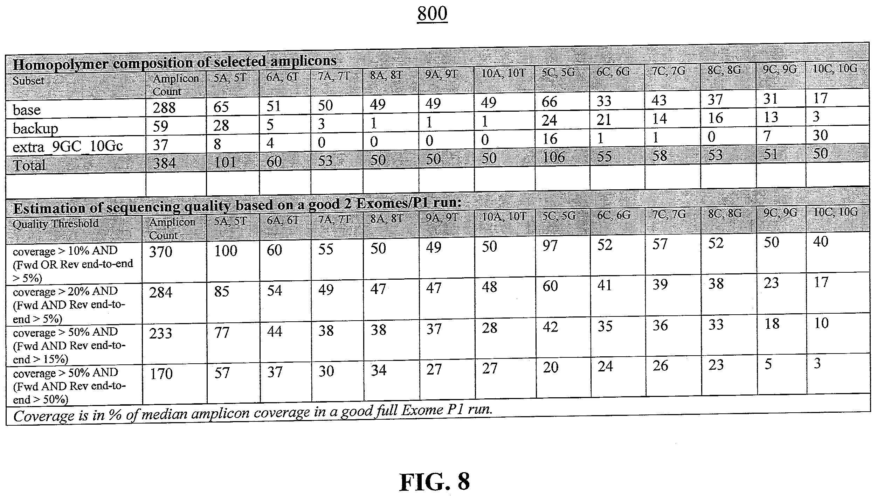

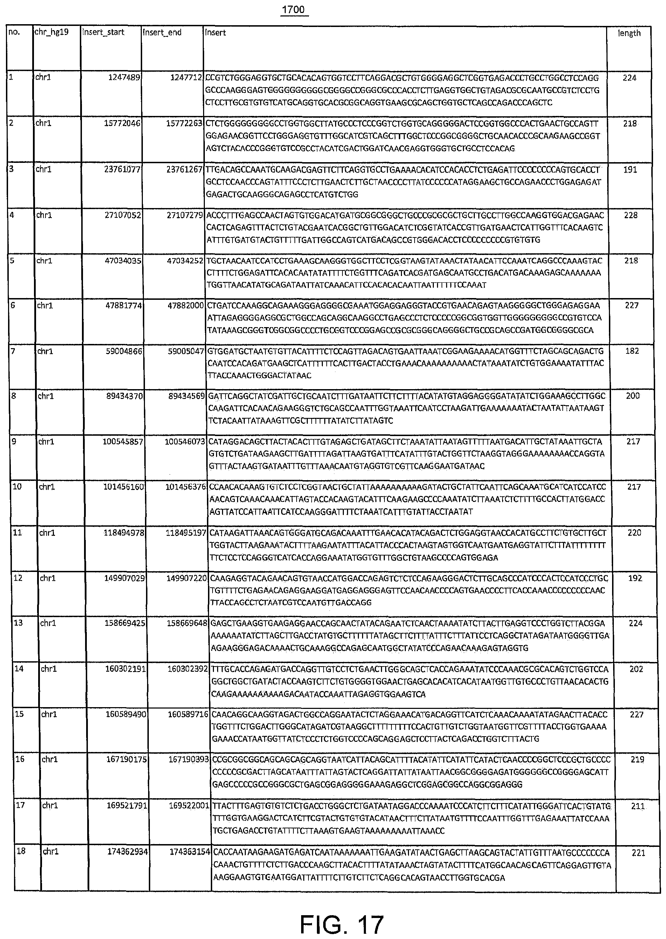

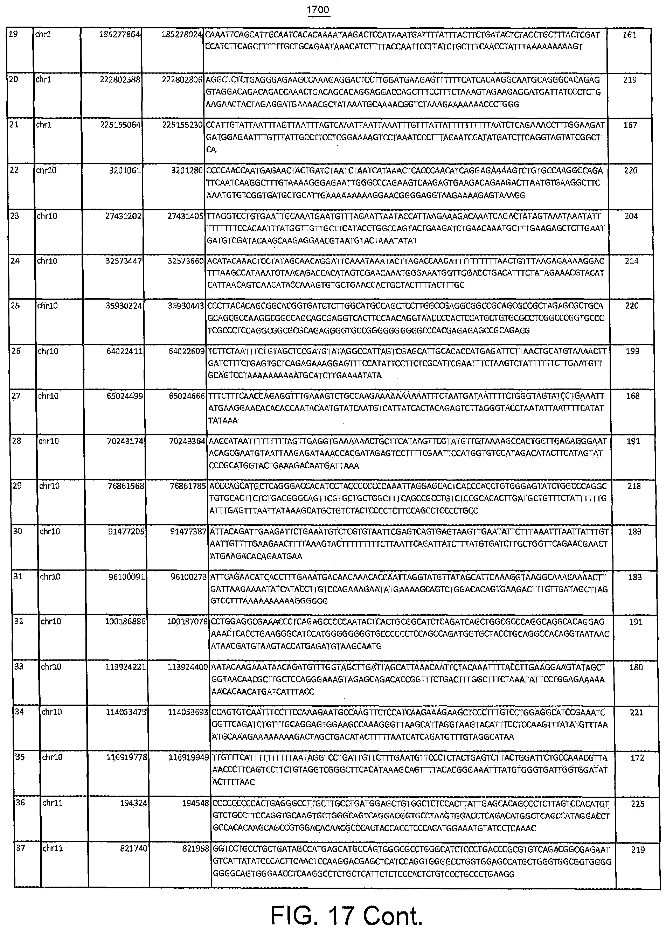

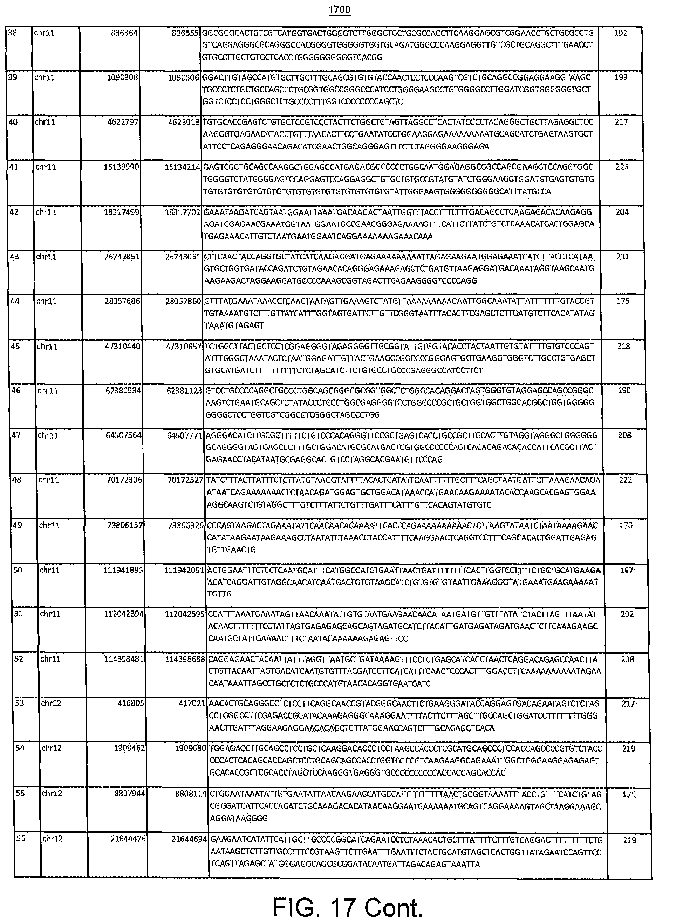

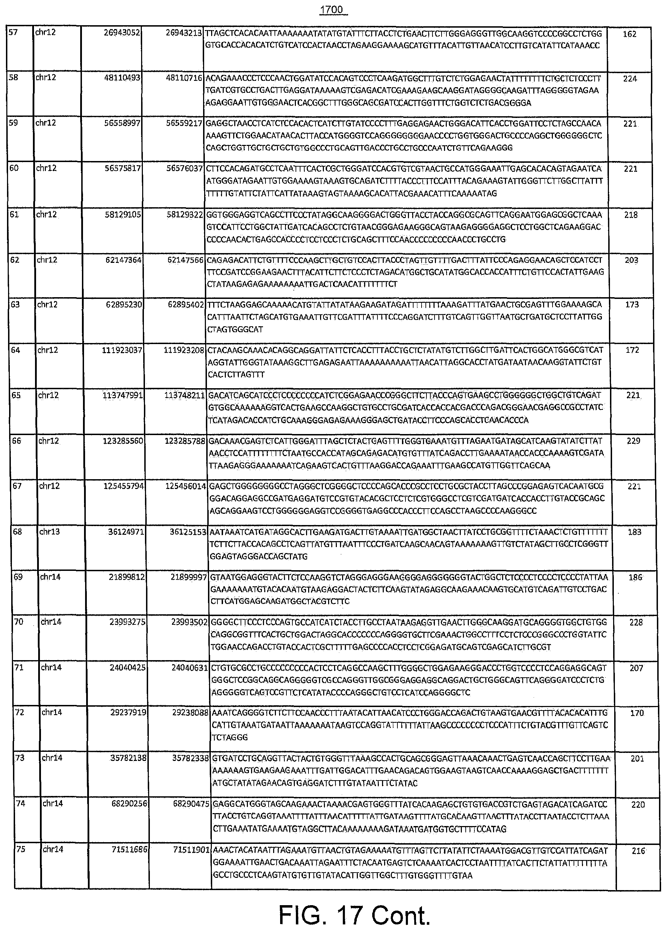

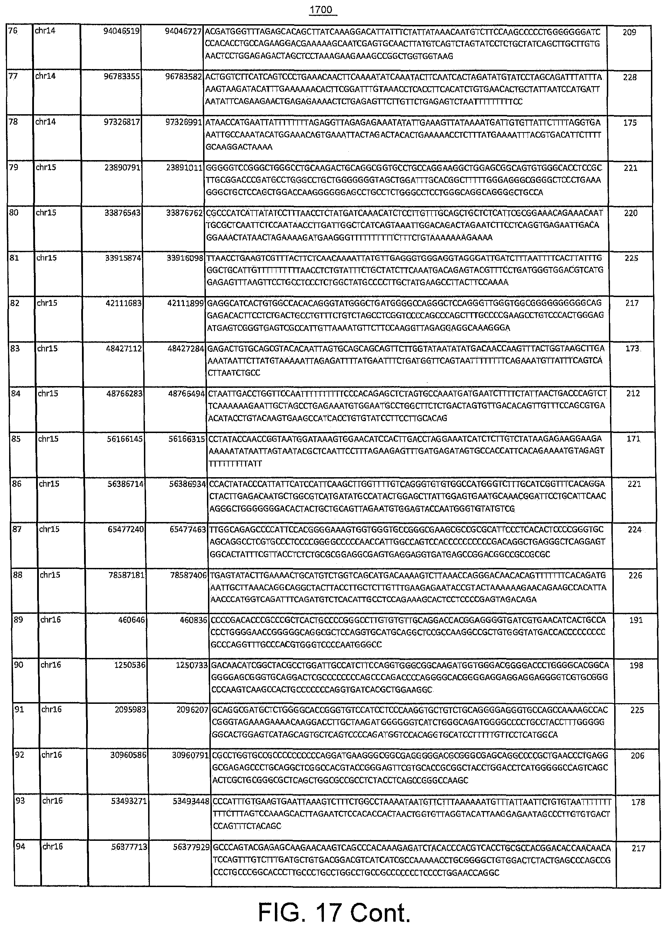

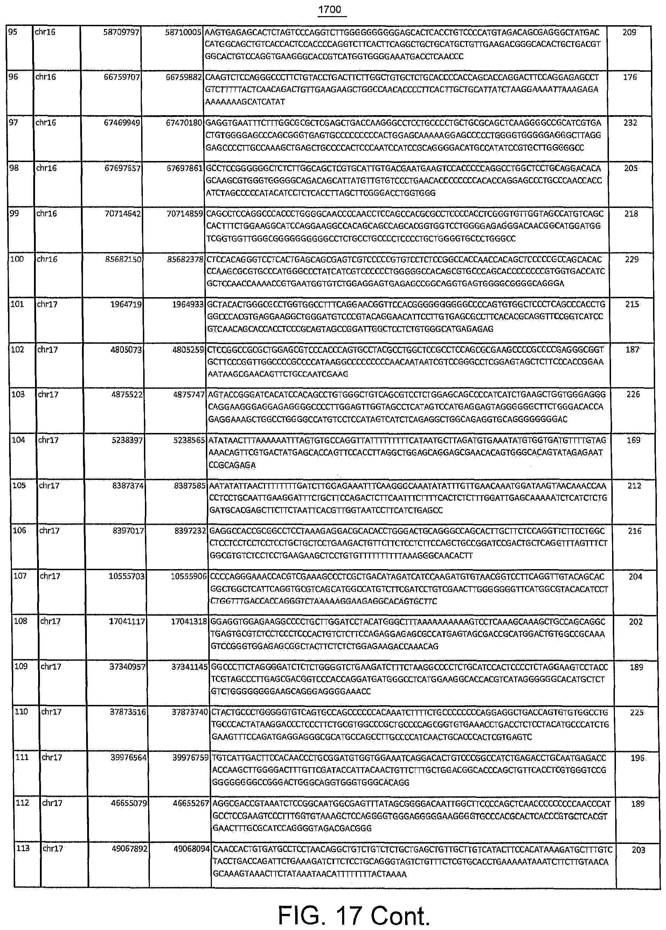

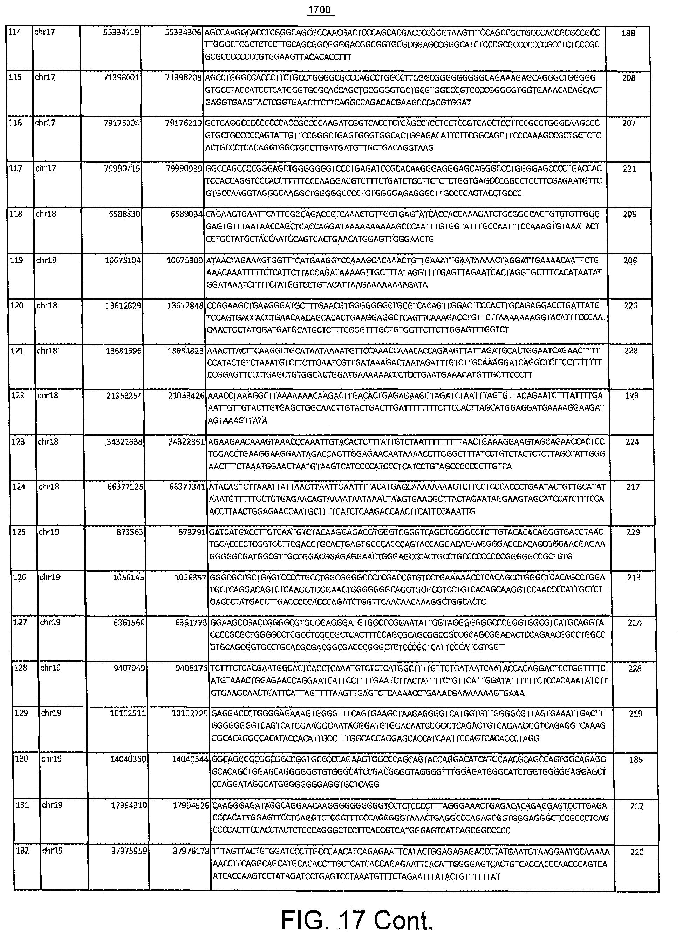

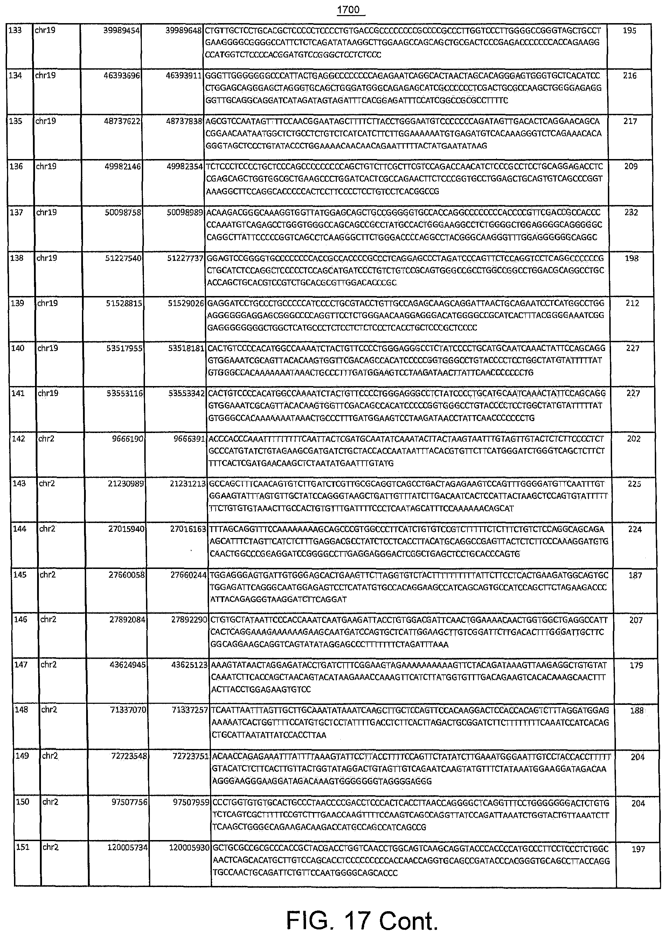

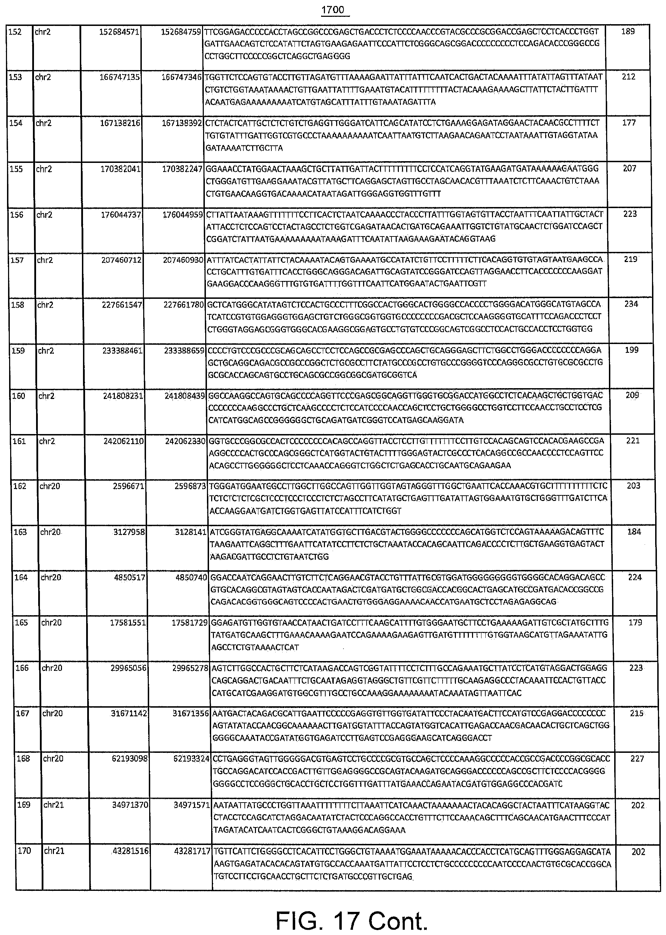

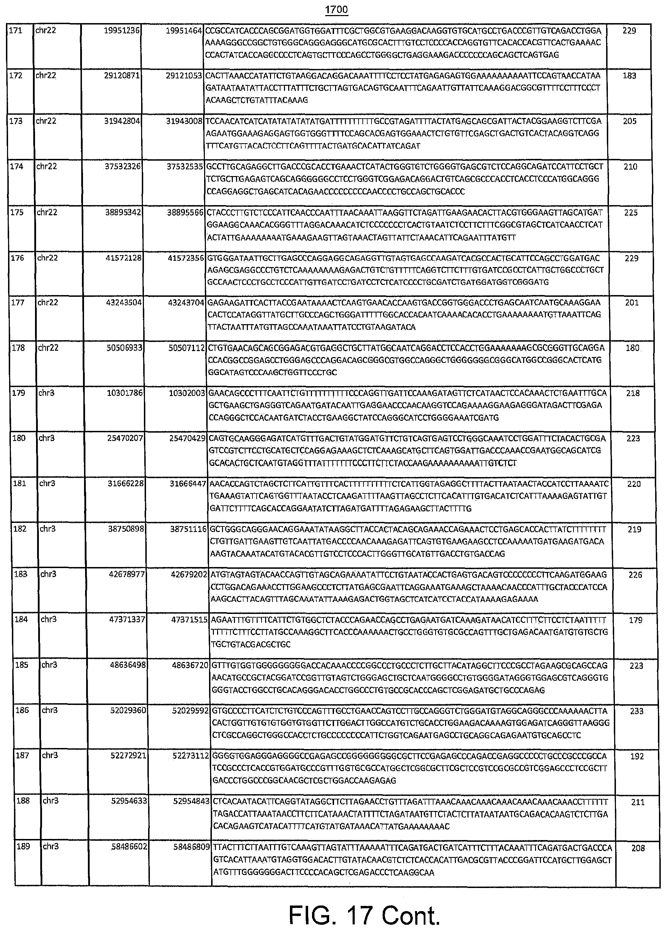

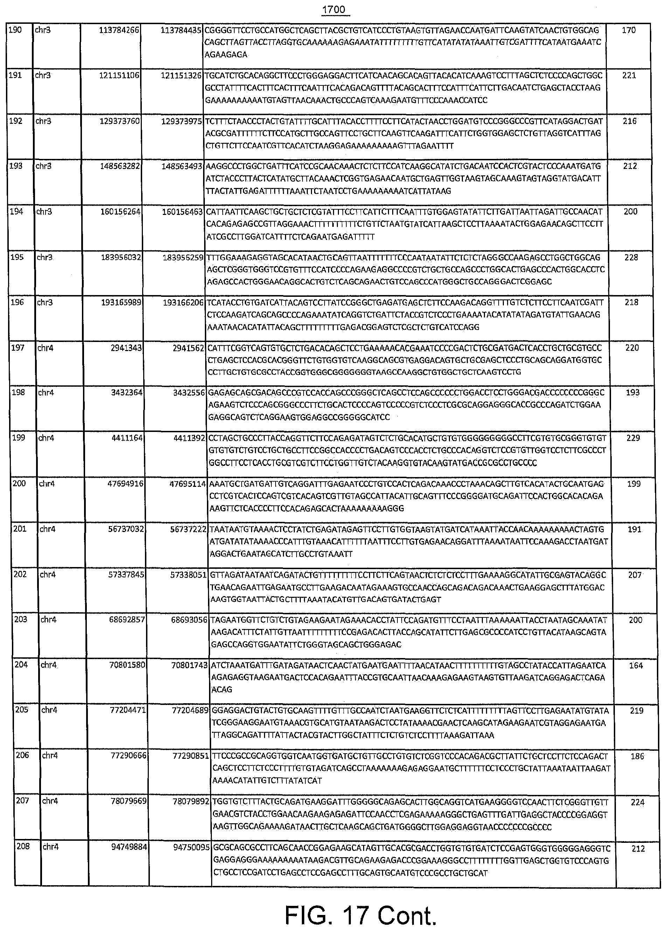

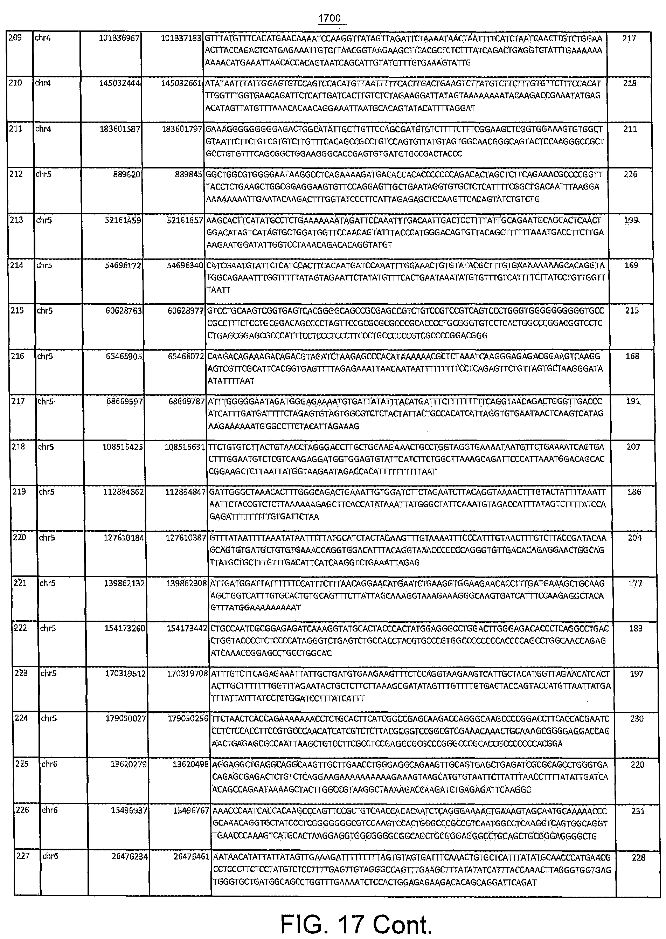

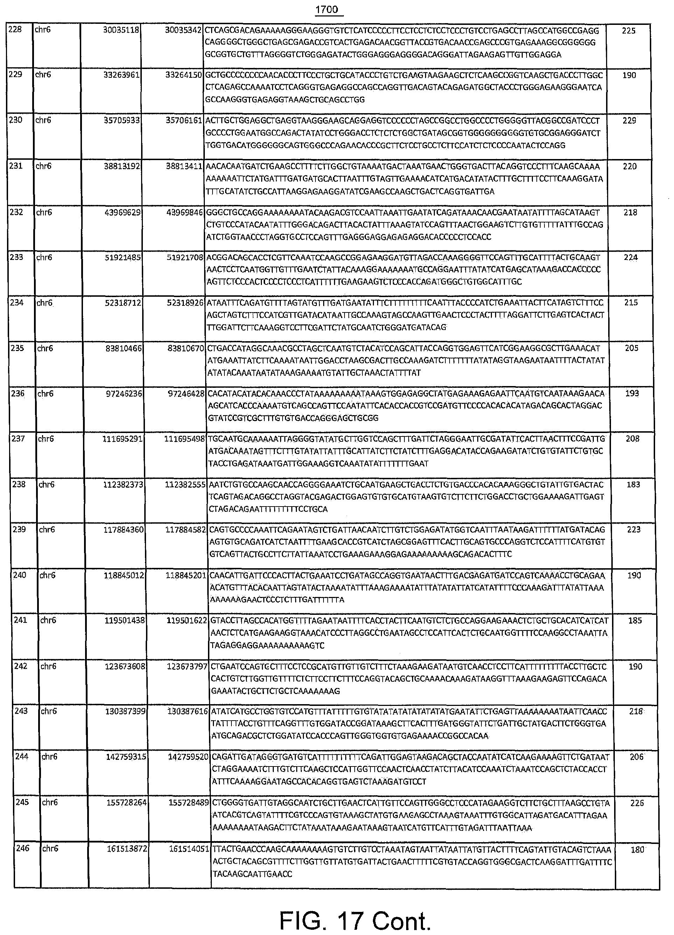

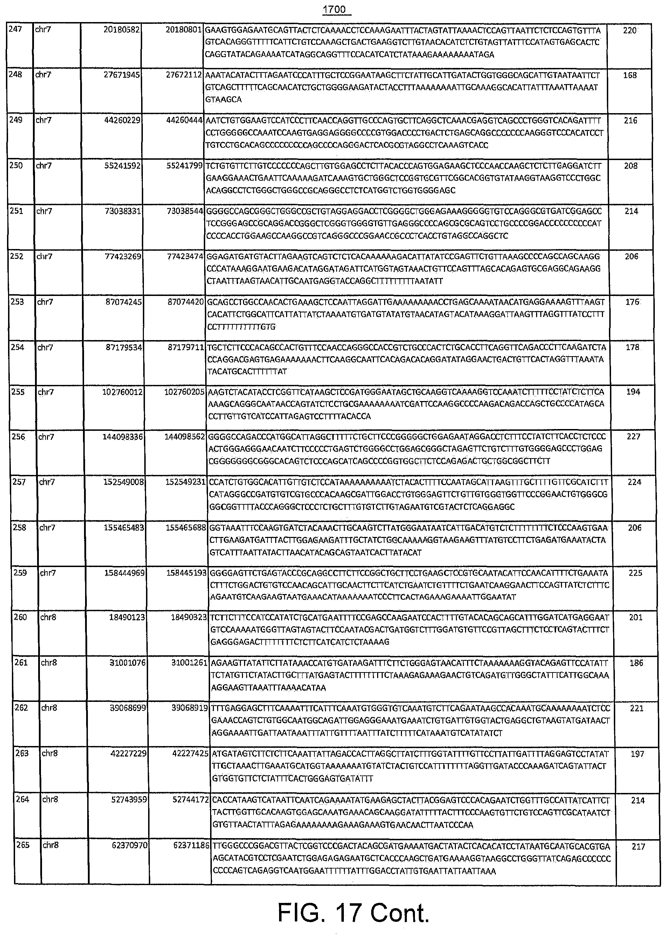

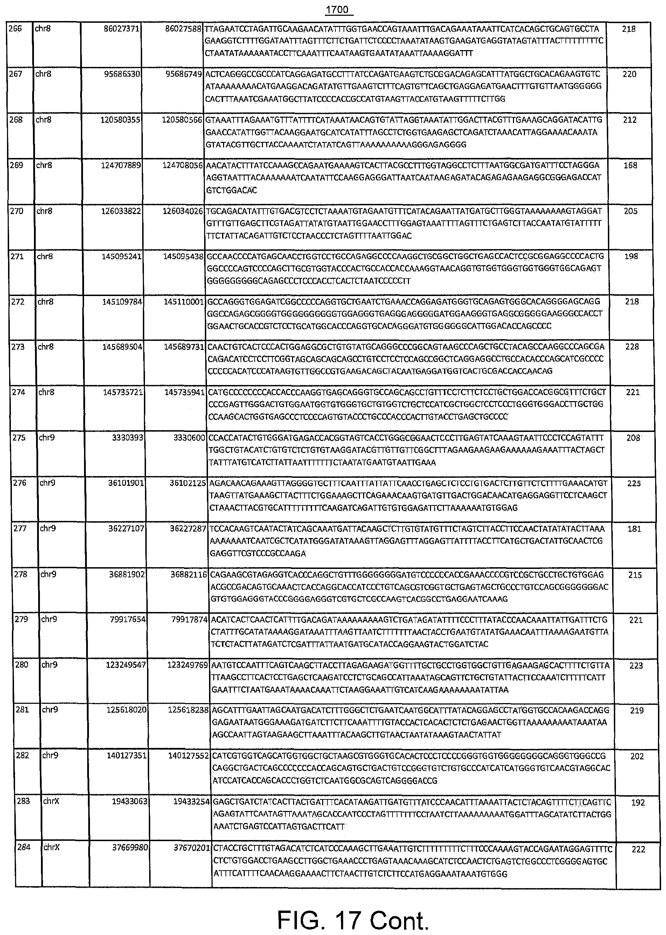

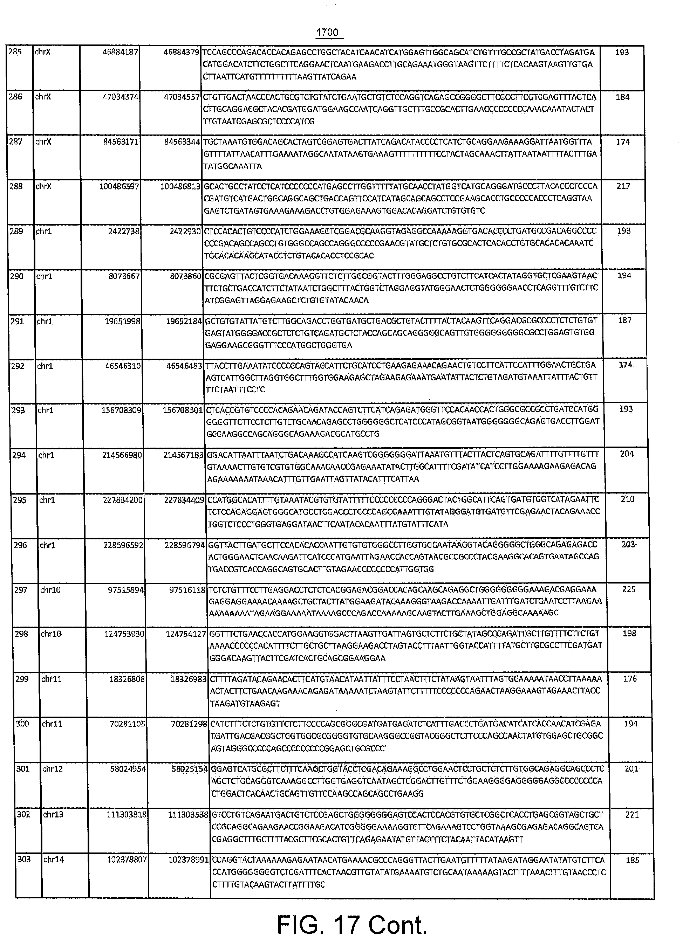

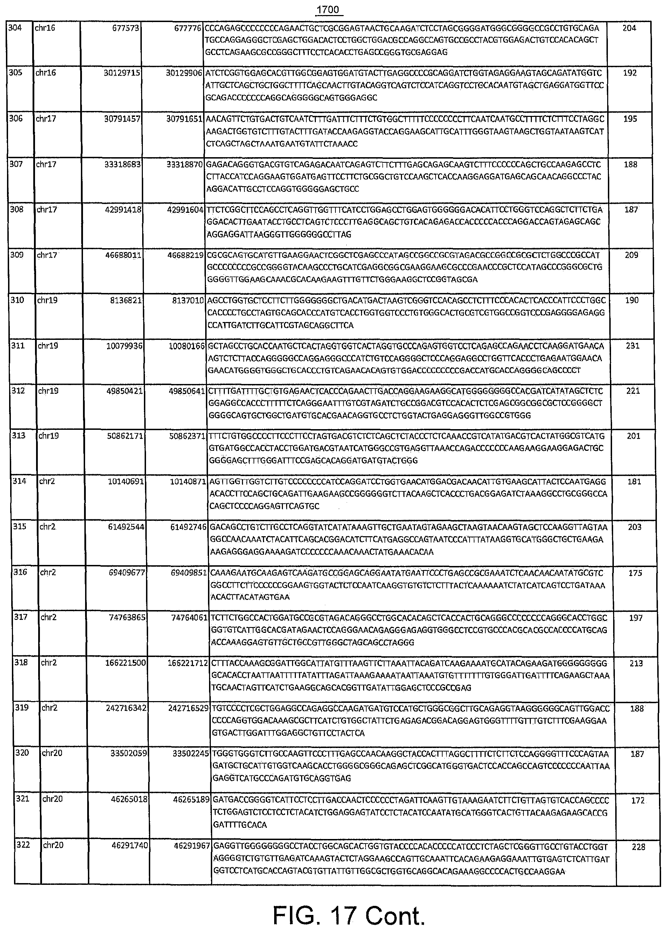

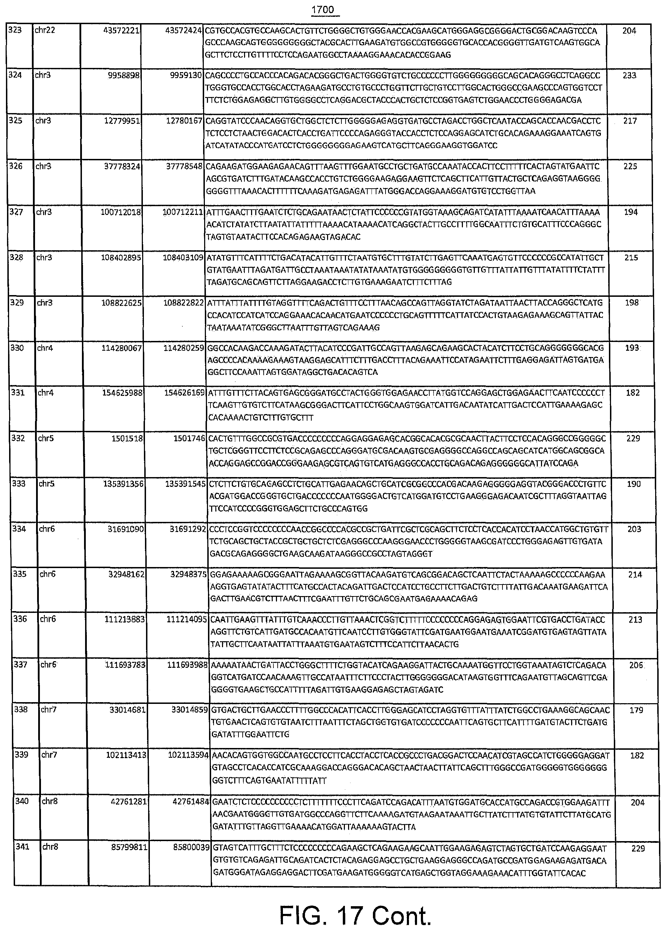

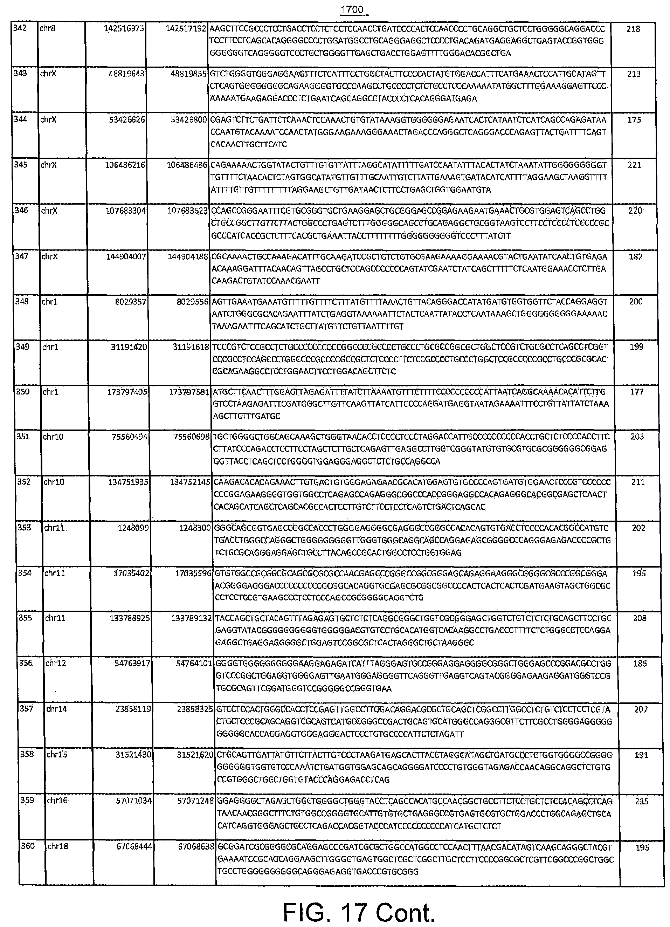

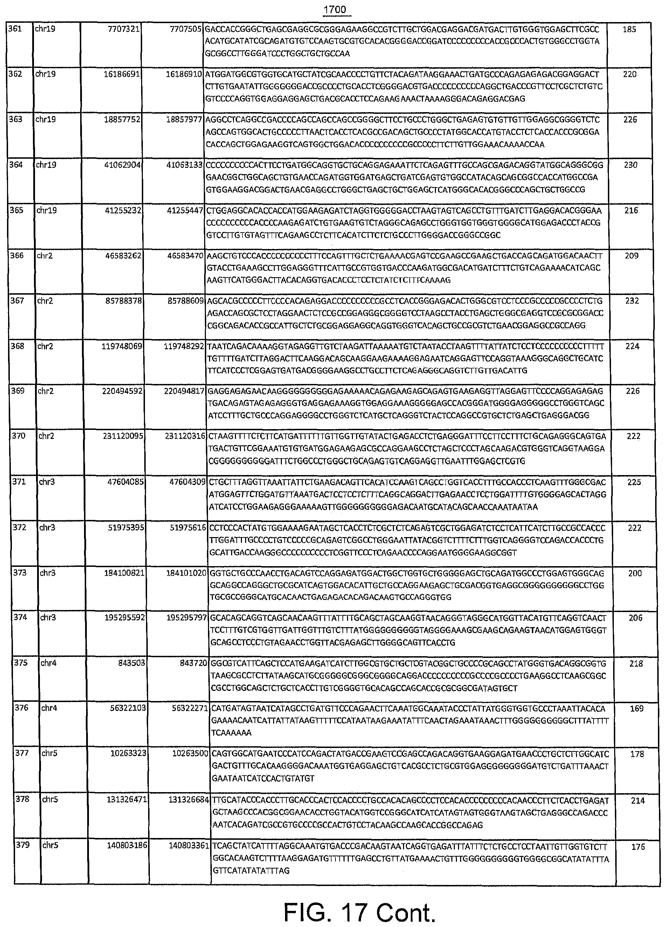

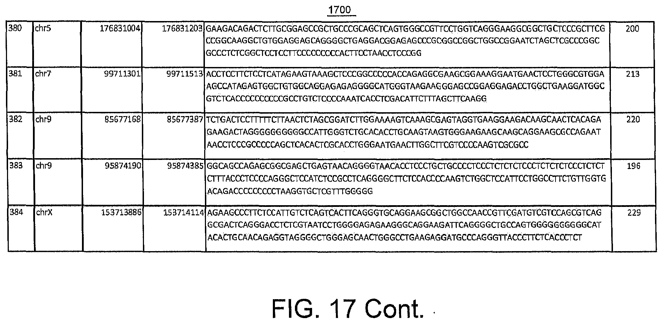

FIG. 8 shows homopolymer representation statistics for an exemplary recalibration panel 800. The upper table includes exemplary homopolymer compositions of amplicons in the exemplary recalibration panel 800. The amplicons shown in the upper table may represent a subset of amplicons on the Ion AmpliSeq^{™} Exome Panel. The "base" group of amplicons may represent selected amplicons that are inside NIST NA12878 high-confidence regions with no variants and reasonably high sequencing quality that further satisfy one or more of the following exemplary constraints: (1) only one n-mer (where n is 6 or more) may be allowed on a given template; (2) a minimal separation of 7 bases between n-mers, where n is 4 or more; and (3) no overlapping between the amplicons and/or no n-mers longer than 10-mers, with a few exceptions (e.g., several G or C n-mers can be on the same strand if they are separated by at least 7 bases and at least 3 bases from A/T n-mers). An exemplary reference genome in addition to NIST NA12878 may include NIST RM 8398. The "backup" group of amplicons may represent additional amplicons with higher sequencing quality (including mostly G/C *n*-mers). The "extra_9GC_10GC" group of amplicons may represent additional amplicons with 9G/9C/10G/10C *n*-mers. The lower table includes an estimation of sequencing quality, e.g., based on a good two-exome run on an Ion Proton^{™} system implementing Ion Torrent^{™} sequencing technology. FIG. 17 includes table 1700, which lists the chromosome, insert start, insert end, and insert for each of the 384 amplicons discussed in FIG. 8. Among these, inserts 1 through 288 may correspond to the "base" group; inserts 289 through 347 may correspond to the "backup" group, and inserts 348 through 384 may correspond to the "extra_9GC_10GC" group.

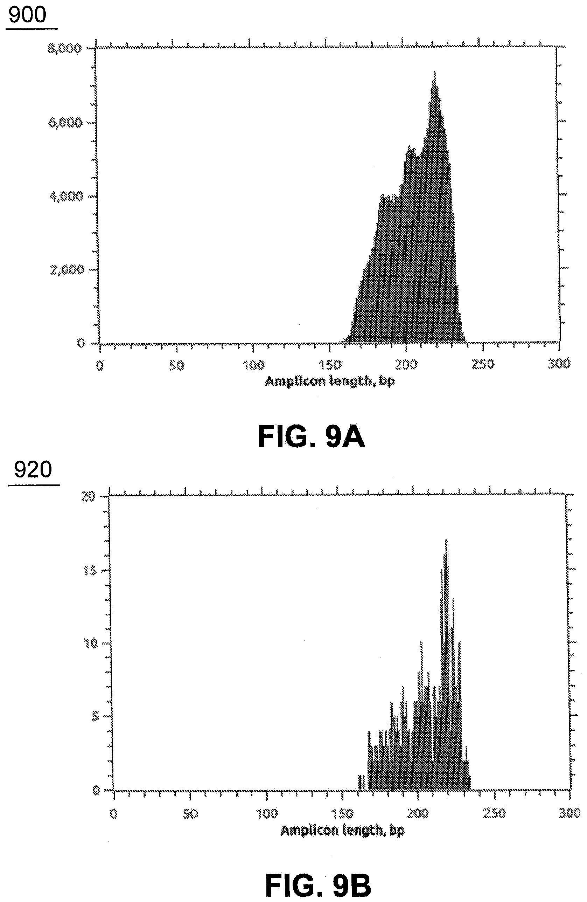

FIGS. 9A and 9B illustrate exemplary distributions of amplicon length for a two-exome sequencing run. FIG. 9A includes an exemplary distribution 900 of amplicon length for a full AmpliSeq^{™} Exome Panel run (e.g., 1/2 of an Ion Proton^{™} system run implementing Ion Torrent^{™} sequencing technology). FIG. 9B includes an exemplary distribution 920 of amplicon length for the subset of 384 amplicons discussed in FIG. 8 and listed in FIG. 17.

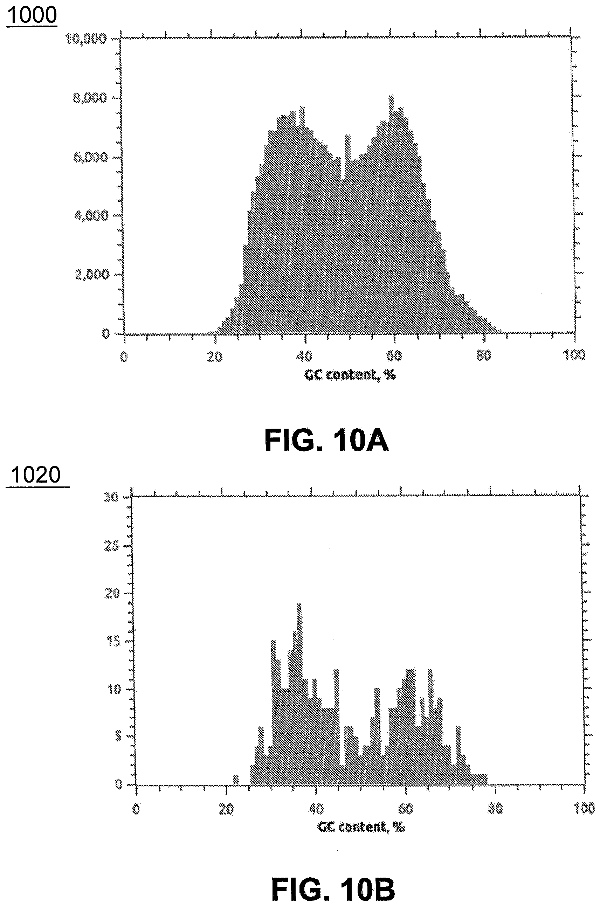

FIGS. 10A and 10B show exemplary distributions of GC content for a two-exome sequencing run. FIG. 10A includes an exemplary distribution 1000 of GC content for the full AmpliSeq^{™} Exome Panel run (e.g., half of an Ion Proton^{™} system run implementing Ion Torrent^{™} sequencing technology). FIG. 10B shows a distribution 1020 of GC content for the subset of 384 amplicons discussed in FIG. 8 and listed in FIG. 17.

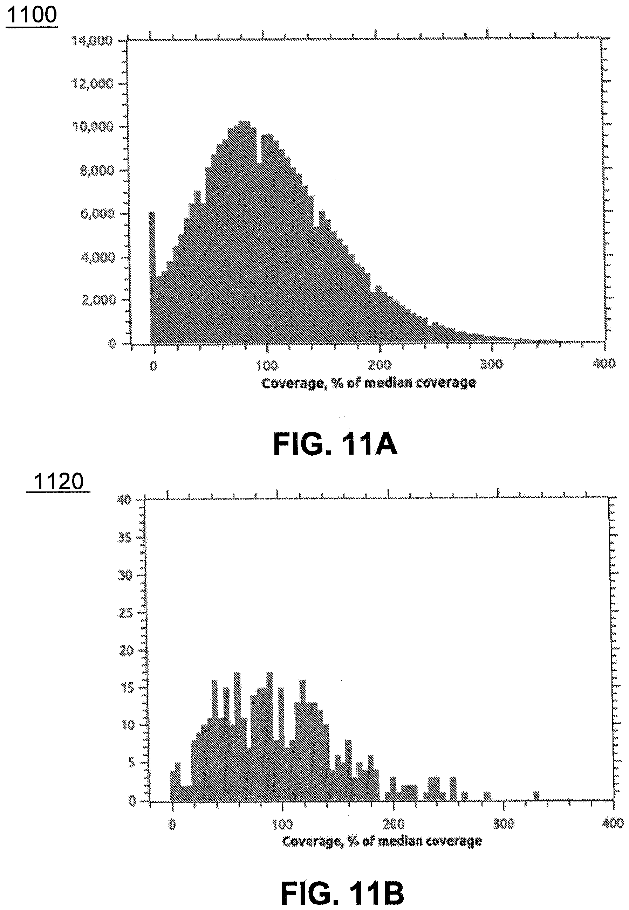

FIGS. 11A and 11B show exemplary distributions of coverage, expressed as percentage of median coverage, for a two-exome sequencing run. FIG. 11A includes an exemplary distribution 1100 of coverage, expressed as percentage of median coverage, for the full AmpliSeq^{™} Exome Panel run (e.g., 1/2 of an Ion Proton^{™} system run implementing Ion Torrent^{™} sequencing technology). FIG. 11B includes an exemplary distribution 1120 of coverage, expressed as percentage of median coverage, for the subset of 384 amplicons discussed in FIG. 8 and listed in FIG. 17. The leftmost peak in FIG. 11B may represent additional 10G/10C *n*-mers.

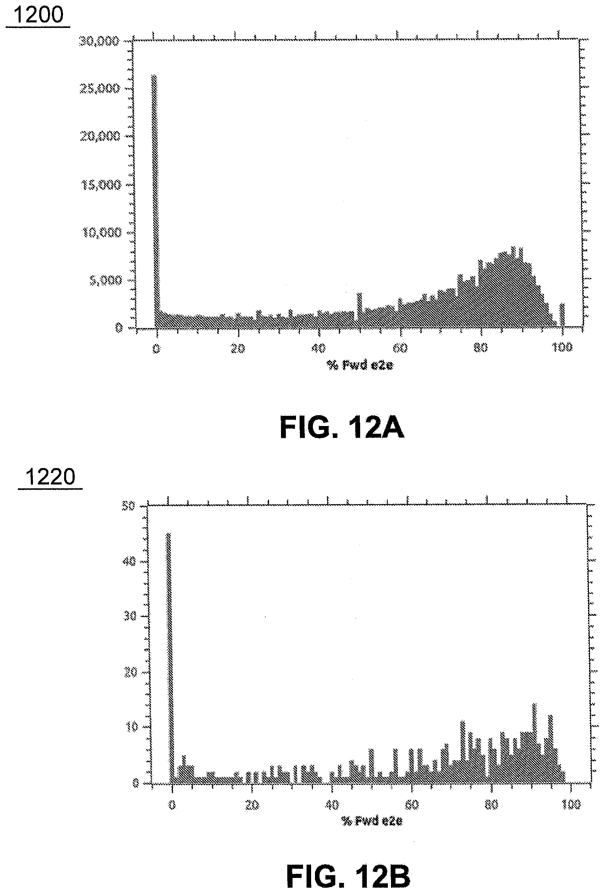

FIGS. 12A and 12B illustrate exemplary distributions of percentage forward end-to-end for a two-exome sequencing run. FIG. 12A includes an exemplary distribution 1200 of percentage forward end-to-end for the full AmpliSeq^{™} Exome Panel run (e.g., half of an Ion Proton^{™} system run implementing Ion Torrent^{™} sequencing technology). FIG. 12B includes an exemplary distribution 1220 of percentage forward end-to-end the subset of 384 amplicons discussed in FIG. 8 and listed in FIG. 17. Similarity in the distributions in FIGS. 12A and 12B may indicate that little or no bias is introduced in the recalibration subset by selecting amplicons that over- or under-perform relative to the full set of AmpliSeq^{™} Exome amplicons.

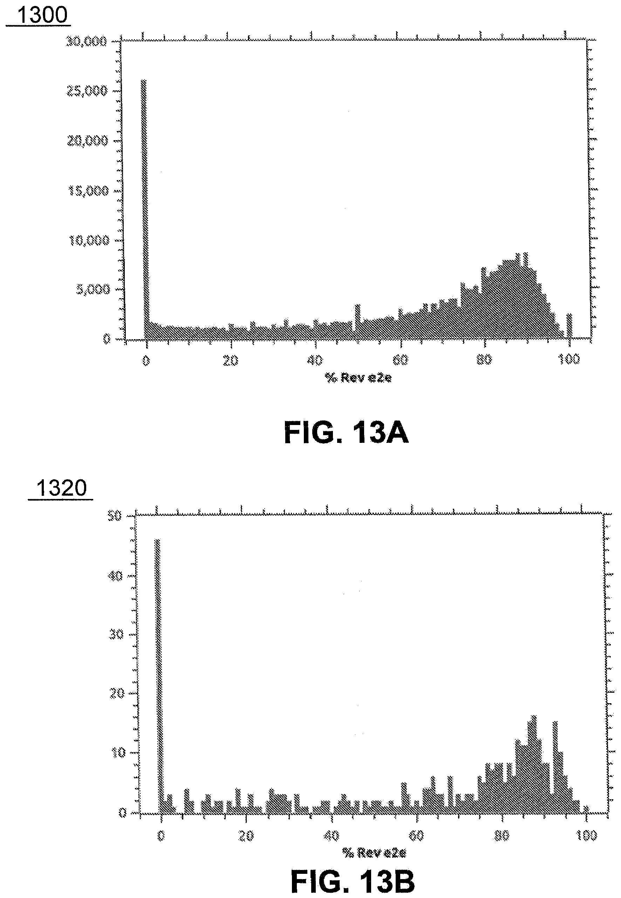

FIGS. 13A and 13B illustrate exemplary distributions of percentage reverse end-to-end for a two-exome sequencing run. FIG. 13A includes an exemplary distribution 1300 of percentage reverse end-to-end for the full AmpliSeq^{™} Exome Panel run (e.g., 1/2 of an Ion Proton^{™} system run implementing Ion Torrent^{™} sequencing technology). FIG. 13B includes an exemplary distribution 1320 of percentage reverse end-to-end for the subset of 384 amplicons discussed in FIG. 8 and listed in FIG. 17. As with FIGS. 12A and 12B, similarities between the distributions in FIGS. 13A and 13B may indicate that little or no bias is introduced in the recalibration subset by selecting amplicons that over- or under-perform relative to full set of AmpliSeq^{™} Exome amplicons.

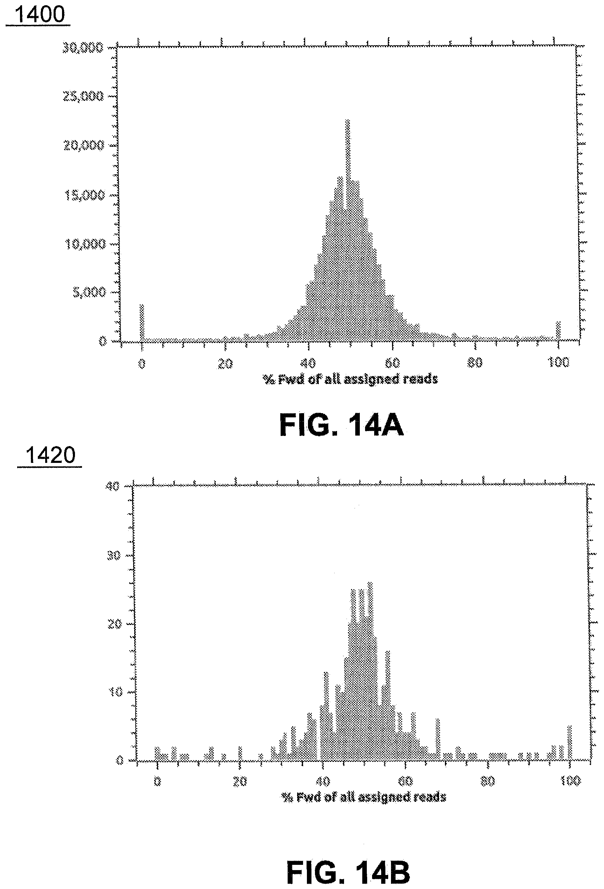

FIGS. 14A and 14B illustrate exemplary distributions of strand bias. FIG. 14A includes an exemplary distribution 1400 of percentage forward of all assigned reads (strand bias) for the full exome run (e.g., 1/2 of an Ion Proton^{™} system run implementing Ion Torrent^{™} sequencing technology). FIG. 14B includes an exemplary distribution 1420 of percentage forward of all assigned reads (strand bias) for a selected subset of recalibration amplicons. Similarity in the distributions in FIGS. 14A and 14B may indicate that little or no bias is introduced in the recalibration subset and that forward and reverse strands may be sequenced equally well (with the exception of a small number of amplicons with long G/C homopolymers).

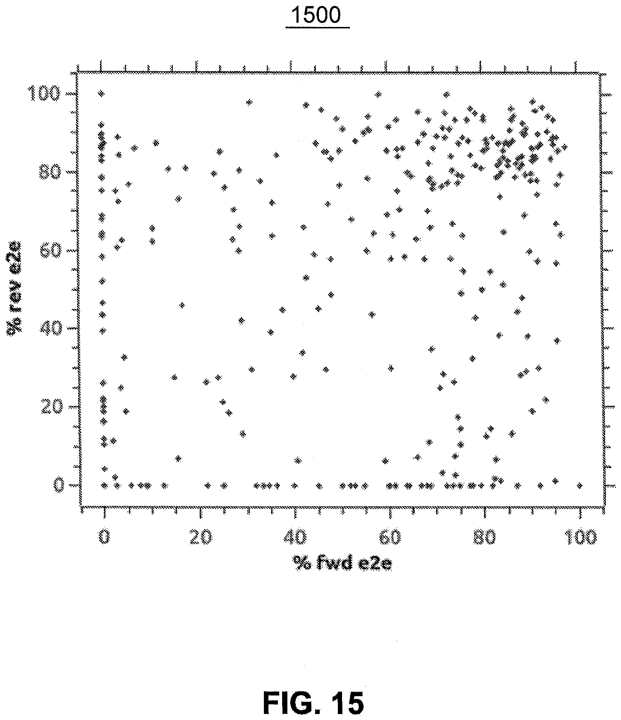

FIG. 15 illustrates an exemplary plot 1500 showing the correlation between forward and reverse end-to-end coverage. The data may pertain to a selected subset of recalibration amplicons. The majority of amplicons may have good end-to-end coverage on both strands. Still, the inclusion of at least some recalibration amplicons of a poorer quality may be desirable to maintain a fair representation (e.g., of typical AmpliSeq^{™} panels) which may include difficult to sequence amplicons (e.g., amplicons with long G/C homopolymers). The area in the bottom left corner of plot 1500 may include 24 poor end-to-end amplicons having a sum of percentage forward end-to-end and percentage reverse end-to-end that is less than 20%.

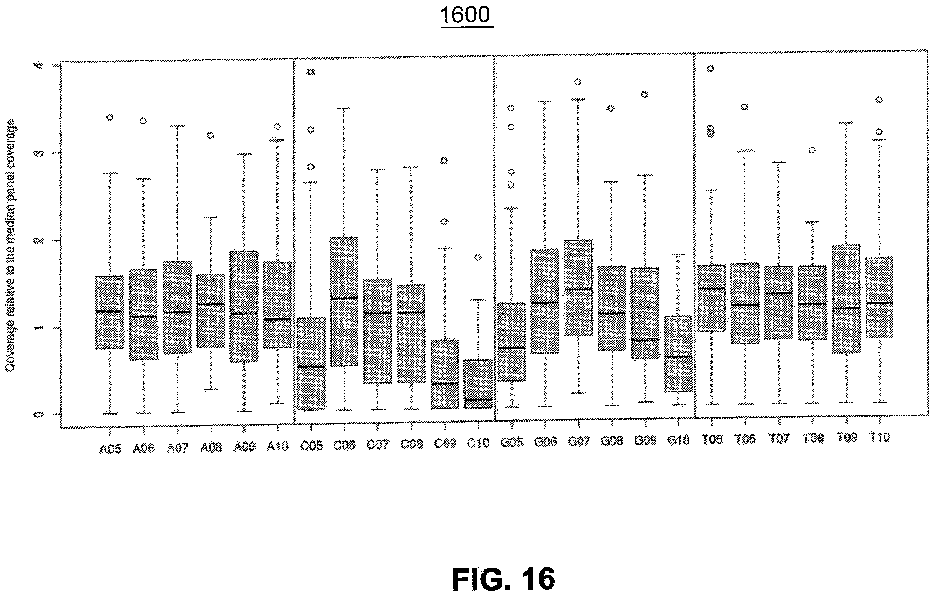

FIG. 16 illustrates an exemplary plot 1600 showing the estimated per-homopolymer coverage relative to the median panel coverage. Here, data may pertain to homopolymers of length 5 through 10 for nucleotide types A, C, G, and T.

In an embodiment, series of measured intensities obtained for panels of amplicons for homopolymer calibration or recalibration as described herein may be used as training subset(s) within the recalibration engine described in Jiang et al., U.S. Pat. Appl. Publ. No. 2014/0316716, published on October 23, 2014, instead of (or in addition to) the series of measured intensities obtained for a randomly selected training subset as described in Jiang et al., U.S. Pat. Appl. Publ. No. 2014/0316716, published on October 23, 2014.

Unless otherwise specifically designated herein, terms, techniques, and symbols of biochemistry, cell biology, genetics, molecular biology, nucleic acid chemistry, nucleic acid sequencing, and organic chemistry used herein follow those of standard treatises and texts in the relevant field.

Although the present description described in detail certain embodiments, other embodiments are also possible. For instance, while described embodiments may include recalibration panels with amplicons that are 200 base pairs in length, the embodiments may also be tailored to recalibration panels with amplicons that are longer in length (e.g., 600 base pairs in length).

## Claims

1. A method for nucleic acid sequencing, comprising:
(a) disposing a plurality of template polynucleotide strands in a plurality of defined spaces of a sensor array, the template polynucleotide strands comprising a predetermined set of homopolymer recalibration template polynucleotide strands;
(b) exposing a plurality of the template polynucleotide strands, including the predetermined set of homopolymer recalibration template polynucleotide strands in the defined spaces, to a series of flows of nucleotide species flowed according to a predetermined ordering; and
(c) determining sequence information for the plurality of the template polynucleotide strands, including the predetermined set of homopolymer recalibration template polynucleotide strands in the defined spaces, based on the flows of nucleotide species, to generate a plurality of sequencing reads corresponding to the template polynucleotide strands,
wherein the predetermined set of homopolymer recalibration template polynucleotide strands comprises amplicon sequences that together comprise at least a predetermined minimal threshold number of homopolymers of each homopolymer length between a predetermined minimal homopolymer length and a predetermined maximal homopolymer length for one or more of homopolymer types A, T, C, and G.

2. The method of claim 1, wherein the minimal threshold number of homopolymers is 10, 25, or 50.

3. The method of claim 1, wherein the predetermined set of homopolymer recalibration template polynucleotide strands comprises amplicon sequences that are comprised in high-confidence regions of a reference genome.

4. The method of claim 1, wherein the predetermined set of homopolymer recalibration template polynucleotide strands comprises amplicon sequences that include at most one homopolymer of length 6, 7, 8, 9, or 10 per amplicon sequence.

5. The method of claim 1, wherein the homopolymer recalibration template polynucleotide strands comprise amplicon sequences having a minimal distance of 7 bases between any homopolymers of length 4, 5, 6, 7, 8, 9, or 10.

6. The method of claim 1, wherein the predetermined set of homopolymer recalibration template polynucleotide strands comprises amplicon sequences that do not overlap.

7. The method of claim 1, wherein the predetermined minimal homopolymer length is 5 and/or wherein the predetermined maximal homopolymer length is 10.

8. A system, including:
a plurality of template polynucleotide strands disposed in a plurality of defined spaces of a sensor array, the template polynucleotide strands comprising a predetermined set of homopolymer recalibration template polynucleotide strands, wherein the predetermined set of homopolymer recalibration template polynucleotide strands comprises amplicon sequences that together comprise at least a predetermined minimal threshold number of homopolymers of each homopolymer length between a predetermined minimal homopolymer length and a predetermined maximal homopolymer length for one or more of homopolymer types A, T, C, and G;
a machine-readable memory; and
a processor configured to execute machine-readable instructions, which, when executed by the processor, cause the system to perform a method for nucleic acid sequencing, comprising:
(a) exposing the plurality of the template polynucleotide strands, including the predetermined set of homopolymer recalibration template polynucleotide strands in the defined spaces, to a series of flows of nucleotide species flowed according to a predetermined ordering; and
(b) determining sequence information for the plurality of the template polynucleotide strands, including the predetermined set of homopolymer recalibration template polynucleotide strands in the defined spaces, based on the flows of nucleotide species, to generate a plurality of sequencing reads corresponding to the template polynucleotide strands.

9. The system of claim 8, wherein the homopolymer recalibration template polynucleotide strands comprise amplicon sequences that are comprised in high-confidence regions of a reference genome with no variants.

10. A method for preparing a homopolymer recalibration panel, comprising:
extracting, from a set of amplicons used in sequencing-by-synthesis, a set of candidate amplicons satisfying a first set of criteria, wherein the first set of criteria includes amplicons known to belong to high-confidence regions of a reference genome with no variants; and
selecting, from the set of candidate amplicons, a reduced set of amplicons satisfying a second set of criteria, wherein the second set of criteria includes selecting amplicons that together comprise at least a minimal threshold number of homopolymers of each
homopolymer length between a predetermined minimal homopolymer length and a predetermined maximal homopolymer length for one or more of homopolymer types A, T, C, and G.

11. The method of claim 10, wherein the minimal threshold number of homopolymers is 10, 25, or 50.

12. The method of claim 10, wherein the predetermined minimal homopolymer length and the predetermined maximal homopolymer length is for each of homopolymer types A, T, C, and G.

13. The method of claim 10, further comprising:
determining underrepresented homopolymers of the set of candidate amplicons; and
augmenting the set of candidate amplicons with a predetermined number of the underrepresented homopolymers.

14. The method of claim 10, wherein the reference genome with no variants is NIST NA12878.

15. The method of claim 10, wherein the reduced set of amplicons comprises at most one homopolymer of length 6, 7, 8, 9, or 10 per amplicon.

16. The method of claim 10, wherein the reduced set of amplicons comprises amplicons having a minimal distance of 7 bases between any homopolymers of length 4, 5, 6, 7, 8, 9, or 10.

17. The method of claim 10, wherein the reduced set of amplicons comprises amplicons that do not overlap.

18. The method of claim 10, wherein the predetermined minimal homopolymer length is 5 and/or wherein the predetermined maximal homopolymer length is 10.

19. The method of claim 10, further comprising:
disposing the reduced set of amplicons in a plurality of defined spaces of a sensor array.

20. The method of claim 10, further comprising:
exposing the reduced set of amplicons to a series of flows of nucleotide species flowed according to a predetermined ordering; and
determining sequence information for the reduced set of amplicons based on the flows of nucleotide species, to generate a plurality of sequencing reads corresponding to the reduced set of amplicons.

## Patentansprüche

1. Verfahren für eine Nukleinsäuresequenzierung, umfassend:
(a) Aufbringen einer Vielzahl von Polynukleotid-Matrizensträngen in einer Vielzahl von definierten Bereichen eines Sensorarrays, wobei die Polynukleotid-Matrizenstränge einen vorbestimmten Satz von Homopolymer-Rekalibrierungs-Polynukleotid-Matrizensträngen umfassen;
(b) Aussetzen einer Vielzahl der Polynukleotid-Matrizenstränge, einschließlich des vorbestimmten Satzes von Homopolymer-Rekalibrierungs-Polynukleotid-Matrizensträngen in den definierten Bereichen, einer Reihe von Flüssen von Nukleotidspezies, die gemäß einer vorbestimmten Reihenfolge fließen; und
(c) Bestimmen von Sequenzinformationen für die Vielzahl der Polynukleotid-Matrizenstränge, einschließlich des vorbestimmten Satzes von Homopolymer-Rekalibrierungs-Polynukleotid-Matrizensträngen in den definierten Räumen, basierend auf den Strömen von Nukleotidspezies, um eine Vielzahl von Sequenzierungslesevorgängen zu erzeugen, die den Polynukleotid-Matrizensträngen entsprechen,
wobei der vorbestimmte Satz von Homopolymer-Rekalibrierungs-Polynukleotid-Matrizensträngen Amplikonsequenzen umfasst, die zusammen mindestens eine vorbestimmte minimale Schwellenwertanzahl von Homopolymeren jeder Homopolymerlänge zwischen einer vorbestimmten minimalen Homopolymerlänge und einer vorbestimmten maximalen Homopolymerlänge für einen oder mehrere von Homopolymer-Typen A, T, C und G umfassen.

2. Verfahren nach Anspruch 1, wobei die minimale Schwellenwertanzahl von Homopolymeren 10, 25 oder 50 ist.

3. Verfahren nach Anspruch 1, wobei der vorbestimmte Satz von Homopolymer-Rekalibrierungs-Polynukleotid-Matrizensträngen Amplikonsequenzen umfasst, die in Bereichen hoher Konfidenz eines Referenzgenoms enthalten sind.

4. Verfahren nach Anspruch 1, wobei der vorbestimmte Satz von Homopolymer-Rekalibrierung-Polynukleotid-Matrizensträngen Amplikonsequenzen umfasst, die höchstens ein Homopolymer der Länge 6, 7, 8, 9 oder 10 pro Amplikonsequenz einschließen.

5. Verfahren nach Anspruch 1, wobei die Homopolymer-Rekalibrierungs-Polynukleotid-Matrizenstränge Amplikonsequenzen umfassen, die einen minimalen Abstand von 7 Basen zwischen beliebigen Homopolymeren der Länge 4, 5, 6, 7, 8, 9 oder 10 aufweisen.

6. Verfahren nach Anspruch 1, wobei der vorbestimmte Satz von Homopolymer-Rekalibrierungs-Polynukleotid-Matrizensträngen Amplikonsequenzen umfasst, die sich nicht überlappen.

7. Verfahren nach Anspruch 1, wobei die vorbestimmte minimale Homopolymerlänge 5 ist und/oder wobei die vorbestimmte maximale Homopolymerlänge 10 ist.

8. System, das einschließt:
eine Vielzahl von Polynukleotid-Matrizensträngen, die in einer Vielzahl von definierten Bereichen eines Sensorarrays aufgebracht sind, wobei die Polynukleotid-Matrizenstränge einen vorbestimmten Satz von Homopolymer-Rekalibrierungs-Polynukleotid-Matrizensträngen umfassen, wobei der vorbestimmte Satz von Homopolymer-Rekalibrierungs-Polynukleotid-Matrizensträngen Amplikonsequenzen umfasst, die zusammen mindestens eine vorbestimmte minimale Schwellenwertanzahl von Homopolymeren jeder Homopolymerlänge zwischen einer vorbestimmten minimalen Homopolymerlänge und einer vorbestimmten maximalen Homopolymerlänge für einen oder mehrere von Homopolymer-Typen A, T, C und G umfassen;
einen maschinenlesbaren Speicher; und
einen Prozessor, der konfiguriert ist, um maschinenlesbare Anweisungen auszuführen, die, wenn sie durch den Prozessor ausgeführt werden, das System veranlassen, ein Verfahren für Nukleinsäuresequenzierung durchzuführen, umfassend:
(a) Aussetzen der Vielzahl der Polynukleotid-Matrizensträngen, einschließlich des vorbestimmten Satzes von Homopolymer-Rekalibrierungs-Polynukleotid-Matrizensträngen in den definierten Räumen, einer Reihe von Strömen von Nukleotidspezies, die gemäß einer vorbestimmten Reihenfolge strömen; und
(b) Bestimmen von Sequenzinformationen für die Vielzahl der Polynukleotid-Matrizenstränge, einschließlich des vorbestimmten Satzes von Homopolymer-Rekalibrierungs-Polynukleotid-Matrizensträngen in den definierten Räumen, basierend auf den Strömen von Nukleotidspezies, um eine Vielzahl von Sequenzierungslesevorgängen zu erzeugen, die den Polynukleotid-Matrizensträngen entsprechen.

9. System nach Anspruch 8, wobei die Homopolymer-Rekalibrierungs-Polynukleotid-Matrizenstränge Amplikonsequenzen umfassen, die in Bereichen hoher Konfidenz eines Referenzgenoms ohne Varianten enthalten sind.

10. Verfahren zum Herstellen einer Homopolymer-Rekalibrierungsplatte, umfassend:
Extrahieren, aus einem Satz von Amplikons, die bei einer Sequenzierungdurch-Synthese verwendet werden, eines Satzes von Kandidaten-Amplikons, der einen ersten Satz von Kriterien erfüllt, wobei der erste Satz von Kriterien Amplikons einschließt, die bekannt sind, zu Bereichen hoher Konfidenz eines Referenzgenoms ohne Varianten zu gehören; und Auswählen, aus dem Satz von Kandidatenamplikons, eines reduzierten Satzes von Amplikons, der einen zweiten Satz von Kriterien erfüllt, wobei der zweite Satz von Kriterien das Auswählen von Amplikons einschließt, die zusammen mindestens eine minimale Schwellenwertanzahl von Homopolymeren jeder Homopolymerlänge zwischen einer vorbestimmten minimalen Homopolymerlänge und einer vorbestimmten maximalen Homopolymerlänge für einen oder mehrere von Homopolymer-Typen A, T, C und G umfassen.

11. Verfahren nach Anspruch 10, wobei die minimale Schwellenwertanzahl von Homopolymeren 10, 25 oder 50 ist.

12. Verfahren nach Anspruch 10, wobei die vorbestimmte minimale Homopolymerlänge und die vorbestimmte maximale Homopolymerlänge für jeden der Homopolymer-Typen A, T, C und G ist.

13. Verfahren nach Anspruch 10, ferner umfassend:
Bestimmen von unterrepräsentierten Homopolymeren des Satzes von Kandidatenampikons; und Erweitern des Satzes von Kandidatenamplikons mit einer vorbestimmten Anzahl der unterrepräsentierten Homopolymere.

14. Verfahren nach Anspruch 10, wobei das Referenzgenom ohne Varianten NIST NA12878 ist.

15. Verfahren nach Anspruch 10, wobei der reduzierte Satz von Amplikons höchstens ein Homopolymer der Länge 6, 7, 8, 9 oder 10 pro Amplikon umfasst.

16. Verfahren nach Anspruch 10, wobei der reduzierte Satz von Amplikons Amplikons umfasst, die einen minimalen Abstand von 7 Basen zwischen beliebigen Homopolymeren der Länge 4, 5, 6, 7, 8, 9 oder 10 aufweisen.

17. Verfahren nach Anspruch 10, wobei der reduzierte Satz von Amplikons Amplikons umfasst, die sich nicht überlappen.

18. Verfahren nach Anspruch 10, wobei die vorbestimmte minimale Homopolymerlänge 5 ist und/oder wobei die vorbestimmte maximale Homopolymerlänge 10 ist.

19. Verfahren nach Anspruch 10, ferner umfassend:
Anordnen des reduzierten Satzes von Amplikons in einer Vielzahl von definierten Räumen eines Sensorarrays.

20. Verfahren nach Anspruch 10, ferner umfassend:
Aussetzen des reduzierten Satzes von Amplikons einer Reihe von Strömen von Nukleotidspezies, die gemäß einer vorbestimmten Reihenfolge strömen; und
Bestimmen von Sequenzinformationen für den reduzierten Satz von Amplikons basierend auf den Strömen von Nukleotidspezies, um eine Vielzahl von Sequenzierungslesevorgängen zu erzeugen, die dem reduzierten Satz von Amplikons entsprechen.

## Revendications

1. Procédé destiné au séquençage d'acides nucléiques, comprenant :
(a) la mise en place d'une pluralité de brins de polynucléotides modèles dans une pluralité d'espaces définis d'un réseau de capteurs, les brins de polynucléotides modèles comprenant un ensemble prédéterminé de brins de polynucléotides modèles homopolymères de recalibrage ;
(b) l'exposition d'une pluralité des brins de polynucléotides modèles, y compris l'ensemble prédéterminé de brins de polynucléotides modèles homopolymères de recalibrage dans les espaces définis, à une série d'écoulements d'espèces de nucléotide s'écoulant selon un ordre prédéterminé ; et
(c) la détermination d'informations de séquence pour la pluralité de brins de polynucléotides modèles, y compris l'ensemble prédéterminé de brins de polynucléotides modèles homopolymères de recalibrage dans les espaces définis, en fonction des écoulements d'espèces de nucléotide, pour générer une pluralité de lectures de séquençage correspondant aux brins de polynucléotides modèles,
dans lequel l'ensemble prédéterminé de brins de polynucléotides modèles homopolymères de recalibrage comprend des séquences d'amplicon qui comprennent ensemble au moins un nombre seuil minimal prédéterminé d'homopolymères de chaque longueur d'homopolymère entre une longueur d'homopolymère minimale prédéterminée et une longueur d'homopolymère maximale prédéterminée pour un ou plusieurs types d'homopolymères A, T, C et G.

2. Procédé selon la revendication 1, dans lequel le nombre seuil minimal d'homopolymères est 10, 25 ou 50.

3. Procédé selon la revendication 1, dans lequel l'ensemble prédéterminé de brins de polynucléotides modèles homopolymères de recalibrage comprend des séquences d'amplicon qui sont comprises dans des régions à haut niveau de confiance d'un génome de référence.

4. Procédé selon la revendication 1, dans lequel l'ensemble prédéterminé de brins de polynucléotides modèles homopolymères de recalibrage comprend des séquences d'amplicon qui comportent au plus un homopolymère de longueur 6, 7, 8, 9 ou 10 par séquence d'amplicon.

5. Procédé selon la revendication 1, dans lequel les brins de polynucléotides modèles homopolymères de recalibrage comprennent des séquences d'amplicon ayant une distance minimale de 7 bases entre de quelconques homopolymères de longueur 4, 5, 6, 7, 8, 9 ou 10.

6. Procédé selon la revendication 1, dans lequel l'ensemble prédéterminé de brins de polynucléotides modèles homopolymères de recalibrage comprend des séquences d'amplicon qui ne se chevauchent pas.

7. Procédé selon la revendication 1, dans lequel la longueur d'homopolymère minimale prédéterminée est 5 et/ou dans lequel la longueur d'homopolymère maximale prédéterminée est 10.

8. Système, comportant :
une pluralité de brins de polynucléotides modèles mis en place dans une pluralité d'espaces définis d'un réseau de capteurs, les brins de polynucléotides modèles comprenant un ensemble prédéterminé de brins de polynucléotides modèles homopolymères de recalibrage, dans lequel l'ensemble prédéterminé de brins de polynucléotides modèles homopolymères de recalibrage comprend des séquences d'amplicon qui comprennent ensemble au moins un nombre seuil minimal prédéterminé d'homopolymères de chaque longueur d'homopolymère entre une longueur d'homopolymère minimale prédéterminée et une longueur d'homopolymère maximale prédéterminée pour un ou plusieurs des types d'homopolymères A, T, C et G ;
une mémoire lisible par machine ; et
un processeur configuré pour exécuter des instructions lisibles par machine, qui, lorsqu'elles sont exécutées par le processeur, amènent le système à mettre en oeuvre un procédé destiné au séquençage d'acides nucléiques, comprenant :
(a) l'exposition de la pluralité des brins de polynucléotides modèles, y compris l'ensemble prédéterminé de brins de polynucléotides modèles homopolymères de recalibrage dans les espaces définis, à une série d'écoulements d'espèces de nucléotide s'écoulant selon un ordre prédéterminé ; et
(b) la détermination d'informations de séquence pour la pluralité de brins de polynucléotides modèles, y compris l'ensemble prédéterminé de brins de polynucléotides modèles homopolymères de recalibrage dans les espaces définis, en fonction des écoulements d'espèces de nucléotide, pour générer une pluralité de lectures de séquençage correspondant aux brins de polynucléotides modèles.

9. Système selon la revendication 8, dans lequel les brins de polynucléotides modèles homopolymères de recalibrage comprennent des séquences d'amplicon qui sont comprises dans des régions à haut niveau de confiance d'un génome de référence dépourvu de variants.

10. Procédé permettant de préparer un panel de recalibrage d'homopolymères, comprenant :
l'extraction, à partir d'un ensemble d'amplicons utilisés dans un séquençage par synthèse, d'un ensemble d'amplicons candidats respectant un premier ensemble de critères, dans lequel le premier ensemble de critères comporte des amplicons connus pour appartenir à des régions à haut niveau de confiance d'un génome de référence dépourvu de variants ; et la sélection, à partir de l'ensemble d'amplicons candidats, d'un ensemble réduit d'amplicons respectant un second ensemble de critères, dans lequel le second ensemble de critères comporte la sélection d'amplicons qui comprennent ensemble au moins un nombre seuil minimal d'homopolymères de chaque
longueur d'homopolymère entre une longueur d'homopolymère minimale prédéterminée et une longueur d'homopolymère maximale prédéterminée pour un ou plusieurs des types d'homopolymères A, T, C et G.

11. Procédé selon la revendication 10, dans lequel le nombre seuil minimal d'homopolymères est 10, 25 ou 50.

12. Procédé selon la revendication 10, dans lequel la longueur d'homopolymère minimale prédéterminée et la longueur d'homopolymère maximale prédéterminée sont pour chacun des types d'homopolymères A, T, C et G.

13. Procédé selon la revendication 10, comprenant en outre :
la détermination d'homopolymères sous-représentés de l'ensemble d'amplicons candidats ; et l'enrichissement de l'ensemble d'amplicons candidats avec un nombre prédéterminé des homopolymères sous-représentés.

14. Procédé selon la revendication 10, dans lequel le génome de référence dépourvu de variants est NIST NA12878.

15. Procédé selon la revendication 10, dans lequel l'ensemble réduit d'amplicons comprend au plus un homopolymère de longueur 6, 7, 8, 9 ou 10 par amplicon.

16. Procédé selon la revendication 10, dans lequel l'ensemble réduit d'amplicons comprend des amplicons ayant une distance minimale de 7 bases entre de quelconques homopolymères de longueur 4, 5, 6, 7, 8, 9 ou 10.

17. Procédé selon la revendication 10, dans lequel l'ensemble réduit d'amplicons comprend des amplicons qui ne se chevauchent pas.

18. Procédé selon la revendication 10, dans lequel la longueur d'homopolymère minimale prédéterminée est 5 et/ou dans lequel la longueur d'homopolymère maximale prédéterminée est 10.

19. Procédé selon la revendication 10, comprenant en outre :
la mise en place de l'ensemble réduit d'amplicons dans une pluralité d'espaces définis d'un réseau de capteurs.

20. Procédé selon la revendication 10, comprenant en outre :
l'exposition de l'ensemble réduit d'amplicons à une série d'écoulement d'espèces de nucléotide s'écoulant selon un ordre prédéterminé ; et
la détermination d'informations de séquence pour l'ensemble réduit d'amplicons en fonction des écoulements d'espèces de nucléotide, pour générer une pluralité de lectures de séquençage correspondant à l'ensemble réduit d'amplicons.
